# EUROPEAN PATENT APPLICATION

(11) **EP 1 533 374 A1**
(43) Date of publication of application: **25.05.2005**
(21) Application number: 03730658.6
(22) Date of filing: 28.05.2003
(51) Int. Cl.: C12N 15/09, C07K 14/47, A61K 38/00, A61P 3/04, A61P 7/10, A61P 9/10, A61P 9/12, A61P 19/10, A61P 25/04, A61P 35/00, A61P 43/00

(54) **NOVEL PEPTIDES HAVING CAMP PRODUCING ACTIVITY**

(30) Priority: 04.06.2002 JP 2002162797
(71) Applicant: Japan Science and Technology Agency, Kawaguchi-shi, Saitama 332-0012 (JP); Japan as represented by president of National Cardiovascular Center, Suita-shi, Osaka 565-8565 (JP)
(72) Inventor: MINAMINO, Naoto, Neyagawa-shi, Osaka 572-0806 (JP); KATAFUCHI, Takeshi, Ibaraki-shi, Osaka 567-0057 (JP)
(74) Representative: Hiebl, Inge, Dr.
(86) International application number: PCT/JP2003/006641
(87) International publication number: WO 2003/102180

(57) **Abstract**

It is intended to provide novel and useful proteins which are expressed in the central nerve system and act on a calcitonin receptor, genes thereof and medicinal compositions containing the same. Peptides having at least the following amino acid sequence: Ser-Cys-Asn-Thr-Ala-Thr-Cys-Met-Thr-His-          10 Arg-Leu-Val-Gly-Leu-Leu-Ser-Arg-Ser-Gly-        20 Ser-Met-Val-Arg-Ser-Asn-Leu-Leu-Pro-Thr-          30 Lys-Met-Gly-Phe-Lys-Val-Phe-Gly 38; or an amino acid sequence derived from the amino acid sequence by deletion, substitution or addition of part of these amino acids and having the following characteristics (1) being expressed in the central nerve system, (2) strongly acting on a calcitonin receptor, and (3) promoting the cAMP-productivity of a cell, and, still preferably, (4) concentrarion-dependently incorporating sodium ion, (5) inhibiting the uptake of calcium ion, and (6) inhibiting cell proliferation; genes encoding these peptides; and medicinal compositions containing the same.

## Description

### Technical Field

The present invention relates to novel and useful peptides, genes encoding these peptides and pharmaceutical compositions comprising the same. More particularly, the present invention relates to peptides having the properties of (1) to (10); (1) being expressed in central nervous system, (2) strongly acting on calcitonin receptors, (3) promoting a cAMP productivity of cells, (4) incorporating sodium ion in a concentration-dependent manner, (5) suppressing the uptake of calcium ion, and (6) suppressing cell proliferation; genes encoding these peptides; and pharmaceutical compositions comprising the same.

### Background Art

Calcitonin, a known peptide, is a peptide hormone comprising 32 amino acids. In mammals, it is secreted from C cell of thyroid gland and has a function of lowering the calcium level in the blood. It has been utilized as therapeutic agents for such as hypercalcemia and metabolic bone diseases. Calcitonin has been recognized as being present in peripheral systems such as thyroid gland but not as being present in central nervous system. Contrary to the above, calcitonin receptors are present also in central nervous system. Such being the case, there has been a demand for revealing the significance of existence as well as the functions of calcitonin receptors in central nervous system.

A calcitonin gene-related peptide (CGRP) is a peptide comprising 37 amino acids produced in such a manner that mRNA is derived from the same gene as calcitonin by a mechanism of alternative splicing. The calcitonin gene-related peptide (CGRP) has also the same function as calcitonin i.e. reducing the calcium concentration in blood. Moreover, it activates calcium ion channels and increases the numbers of acetylcholine receptors, suggesting the possibility of being a neurotransmitter. It has been reported that the CGRP is localized mostly to centripetal sensory nerve and central nerve (Goodman, et al., *Life Sci.* 38:2169-2178 (1986); Poyner, D. R. *Pharmac. Ther.* 56:23-51 (1992)), and mainly has a role to activate adenylyl cyclase. A wide variety of CGRP functions including dilating peripheral and cerebral blood vessel (Brain, et al., *Nature* 313:54-568 (1985)), increasing heart rate (Sigrist, et al., *Endocrinology* 119:381-389 (1986)), adjusting calcium metabolism (Grunditz, et al., *Endocrinology* 119:2313-2324 (1986)), leading to less intestinal movement (Fargeas, et al., *Peptides* 6:1167-1171 (1985)), regulating glucose metabolism (lowering insulin secretion and insulin sensitivity) (Hermansen, et al., *Reg. Peptides* 27:149-157 (1990)), suppressing the appetite (Molina, et al., *Diabetes* 39:260-265 (1990)) and suppressing growth hormone release (Tannenbaum, et al., *Endocrinology,* 116: 2685-2687 (1985)) have been reported.

CGRP receptors also have already been known to be located in cerebral, cardiovascular system, endothelial and smooth muscle cells.

There also have been reported the inventions relating to the synthetic calcitonin receptor-binding compound characterized by having a molecular weight of less than about 10,000 daltons and forming a reagent covalently linked to a radioactive metal chelating agent, where the reagent has a binding affinity to calcitonin receptor higher than the binding affinity of radioiodated natural calcitonin to the calcitonin receptor (Published Japanese translation of PCT application No. 2001/523,257); the calcitonin receptor activating substance comprising a pyridine derivatives (Unexamined Patent Application Publication No. 2001/294,574); the polynucleotide and polypeptide of human calcitonin gene-related peptide receptor component factor (CGRP-RCF) (Unexamined Patent Application Publication No. 10/201,482), etc.

### Disclosure of the Invention

Those peptides not having been confirmed as acting directly on calcitonin receptors of central nervous system, there has been a demand for a peptide which acts directly and more strongly on calcitonin receptors in central nervous system.

The present invention is targeting to provide novel and useful peptides which being expressed in central nervous system and act on calcitonin receptors, genes encoding these peptides and pharmaceutical compositions containing the same.

The present inventors have been searching novel physiologically active peptides by using the productivity of cAMP from central nervous system as an index and consequently isolated and purified novel peptides believed to act on cells via calcitonin receptors. The sequence of such a peptide structure has been confirmed to be novel from the database search results (Genbank, swissprot, DDBJ). The present inventors named the novel peptides as calcitonin receptor-stimulating peptides (CRSP) as they act on calcitonin receptors.

Accordingly, the present invention relates to a peptide which has at least the following amino acid sequence Ser-Cys-Asn-Thr-Ala-Thr-Cys-Met-Thr-His-          10 Arg-Leu-Val-Gly-Leu-Leu-Ser-Arg-Ser-Gly-          20 Ser-Met-Val-Arg-Ser-Asn-Leu-Leu-Pro-Thr-          30 Lys-Met-Gly-Phe-Lys-Val-Phe-Gly 38 , or an amino acid sequence where a part of amino acids are deleted, substituted or added, and has the following characteristics: (1) being expressed in central nervous system, (2) strongly acting on calcitonin receptors and (3) promoting a cAMP productivity of cells, more preferably, (4) incorporating sodiumion in a concentration-depending manner, (5) suppressing the uptake of calcium ion, and (6) suppressing cell proliferation.

To be more specific, the present invention relates to the above described peptide having an amino acid sequence set forth in SEQ ID NO: 1, SEQ NO: 2, SEQ ID NO: 6, SEQ ID NO: 9, SEQ ID NO: 12, SEQ ID NO: 16 or SEQ ID NO: 19 of the Sequence Listing, or having an amino acid sequence where a part of amino acids are deleted, substituted or added, and having 75% or more homology to the said amino acid sequence.

In another embodiment, the peptides of the present invention comprise peptides having homology to a calcitonin gene-related peptide (CGRP), therefore, they may be expected to have the physiological activity similar to that of CGRP in addition to the effects of CRSP. Accordingly, in addition to the above (1) to (6), the peptides of the invention may also be expected to have the following characteristics similar to those of CGRP: (7) strongly acting on calcitonin-gene related peptide receptors, (8) inducing relaxation of blood vessels, (9) promoting diuresis, and (10) altering the proliferation potency of vascular endothelial cells, vascular smooth muscles and fibroblasts.

The present invention further relates to the genes having base sequences encoding the above described peptides of the invention. To be more specific, the present invention relates to the above described genes having a base sequence set forth in SEQ ID NO: 3, SEQ ID NO: 7, SEQ ID NO: 10, SEQ ID NO: 13, SEQ ID NO: 17 or SEQ ID NO: 20 of the Sequence Listing.

The present invention still further relates to a pharmaceutical composition comprising at least one of the above described peptides of the invention and a pharmaceutically acceptable carrier. Particularly, the present invention relates to the above described pharmaceutical composition where the pharmaceutical composition is a preventive/treating agent for osteoporosis, a preventive/treating agent for cancer, diuretic agent, an appetite suppressant, an analgesic agent, an antihypertensive agent or a drug for prevention of restenosis after PTCA (percutaneous transluminal coronary angioplasty).

### Brief Description of the Drawings

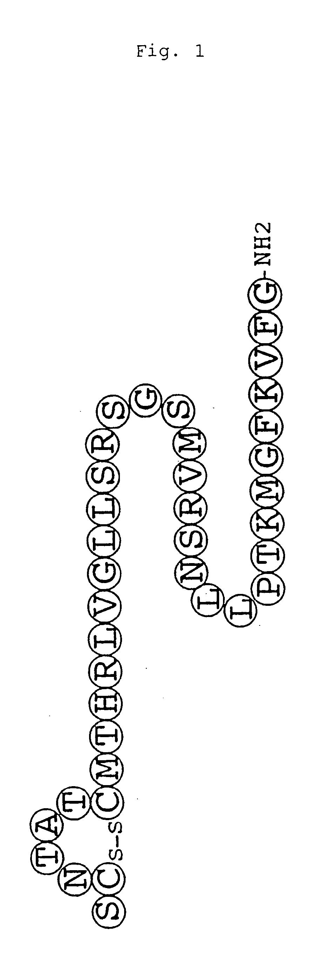

Fig. 1 schematically shows the structure of CRSP of the present invention.

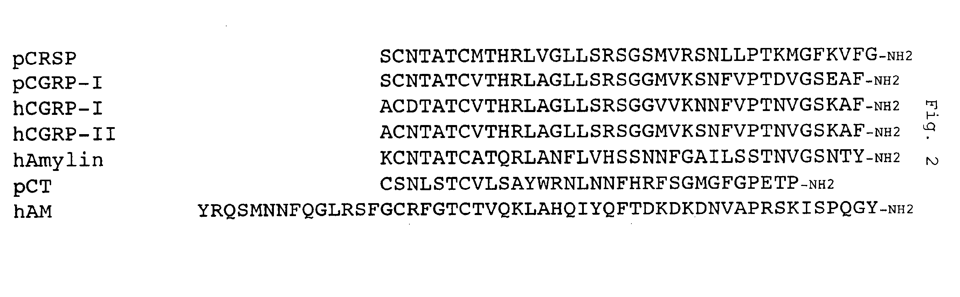

Fig. 2 shows comparisons of the amino acid sequence of CRSP (pCRSP) of the present invention with amino acid sequences of pig calcitonin gene-related peptide (pCGRP-I), human calcitonin gene-related peptide I (hCGRP-I), human calcitonin gene-related peptide II (hCGRP-II), human amylin (hAmylin), pig calcitonin (pCT) and human adrenomedullin (hAM).

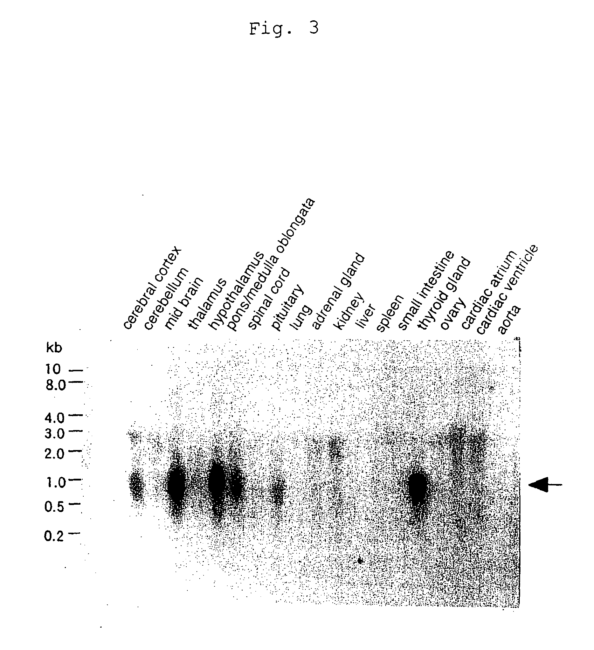

Fig. 3 is a photograph as a drawing which shows the result of Northern blot in search for expression site of CRSP of the present invention. An arrow in Fig. 3 shows the positions of CRSP mRNA.

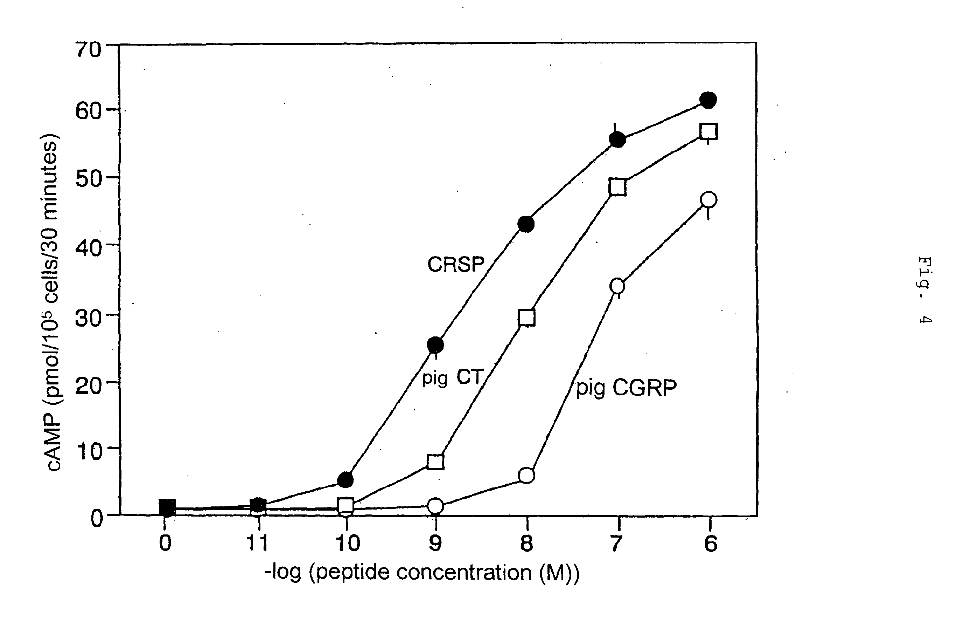

Fig. 4 shows the result of the investigation on a cAMP productivity of CRSP of the present invention using LLC-PK₁ cells. The vertical axis in Fig. 4 shows the production amount of cAMP (pmol/10⁵ cells/30 minutes), while the horizontal axis shows a reverse logarithm (-log (peptide concentration(M))) of concentration of each peptide. A closed circle (•) in Fig. 4 shows CRSP of the present invention, an open circle (○) shows pig calcitonin gene-related peptide (pig CGRP) and an open rhombus (◇) shows pig calcitonin (pig CT).

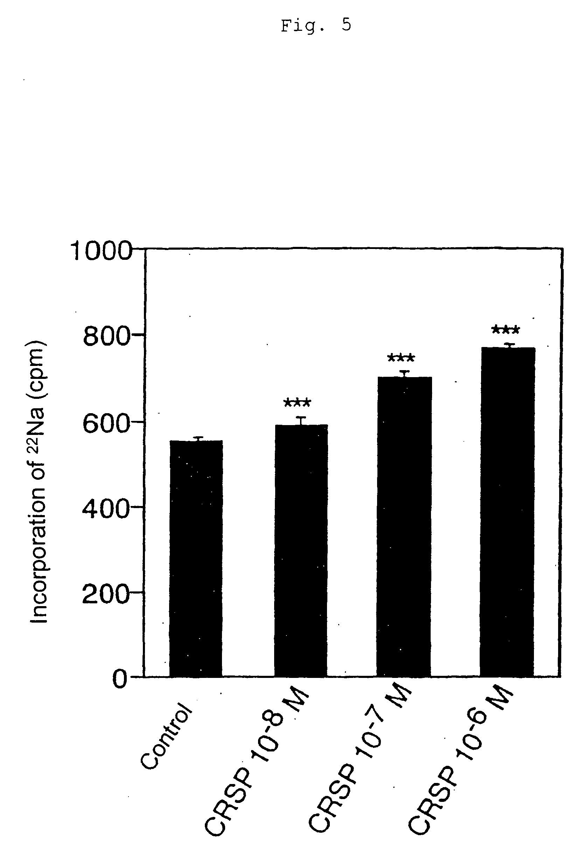

Fig. 5 shows the result of the incorporation test for sodium ion in the absence of 5-(N-ethyl-N-isopropyl) -amiloride (EIPA) by CRSP of the present invention using LLC-PK₁ cells. The vertical axis in Fig. 5 shows incorporated amount (cpm) of each ion, while the left end of the horizontal axis shows control (no peptide added) and to the right thereof shows CRSP of each concentration. The mark *** shows that there was a significant difference when p < 0.001.

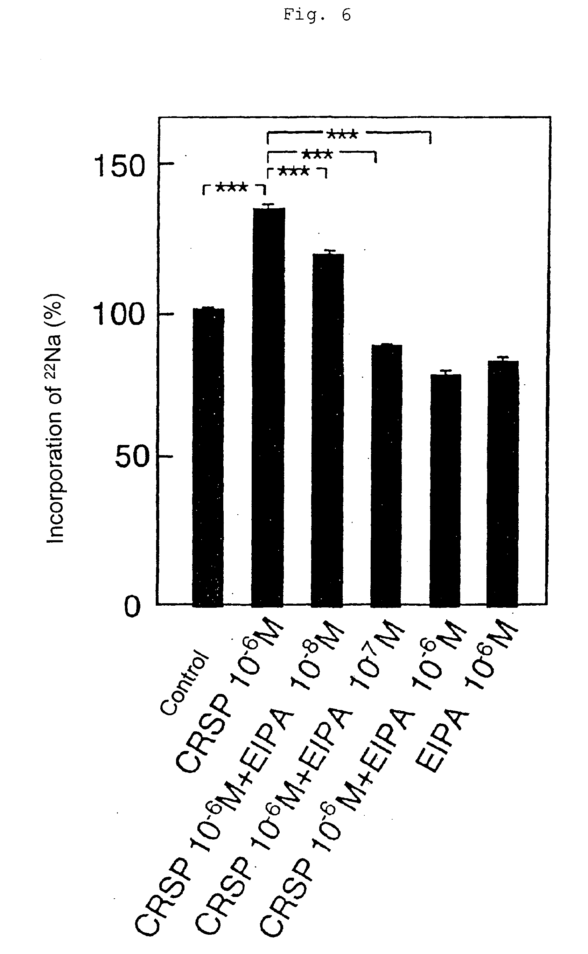

Fig. 6 shows the result of the incorporation for sodium ion in the presence of EIPA by CRSP of the present invention using LLC-PK₁ cells. The vertical axis in Fig. 6 shows incorporated amount (cpm) of each ion, while the left end of the horizontal axis shows control (no peptide added) and to the right thereof shows CRSP and EIPA of each concentration. The mark *** shows that there was a significant difference when p < 0.001.

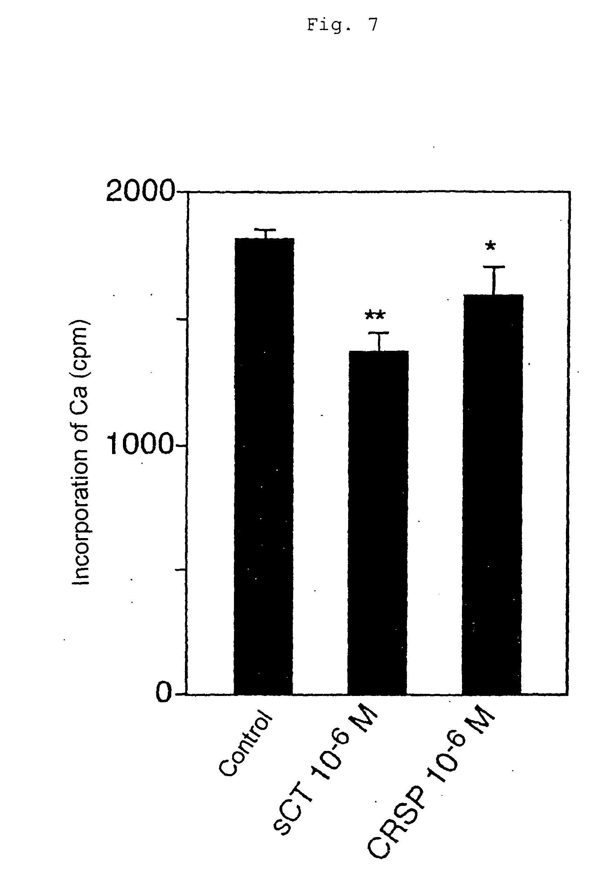

Fig. 7 shows the result of the incorporation for calcium ion by CRSP of the present invention using LLC-PK₁ cells. The vertical axis in Fig. 7 shows incorporated amount (cpm) of each ion, while the left end of the horizontal axis shows control (no peptide added) and to the right thereof shows sCT (salmon calcitonin) and CRSP. The marks * and ** show that there were significant differences when p < 0. 05 and p < 0. 01, respectively.

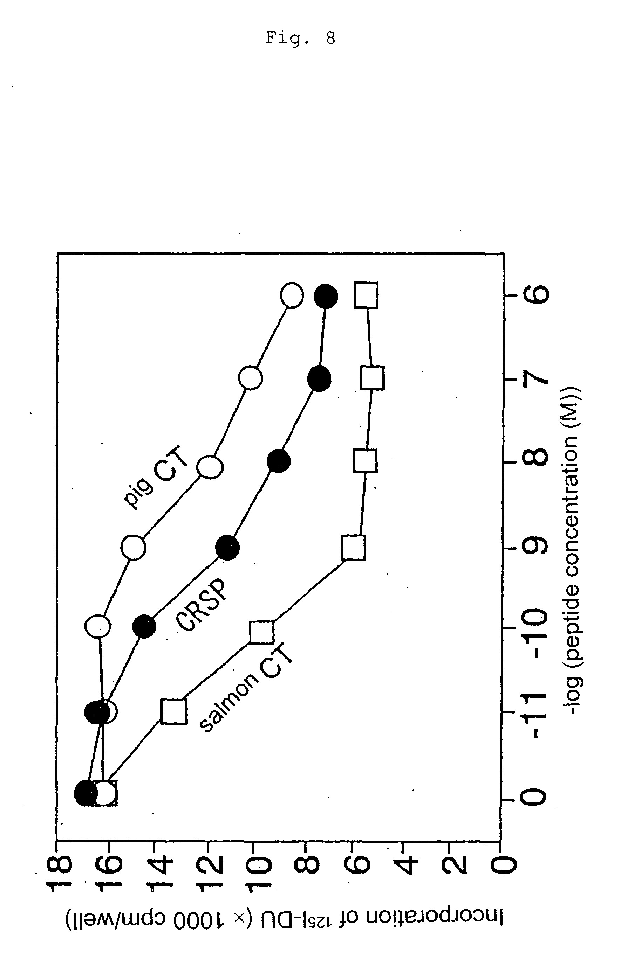

Fig. 8 shows the result of measurement of DNA amount synthesized as a result of cell proliferation by CRSP of the present invention in LLC-PK₁ cells. The vertical axis in Fig. 8 shows incorporated amount of ¹²⁵I-DU (× 100 cpm/well) while the horizontal axis shows a reverse logarithm (-log (peptide concentration (M)) of each peptide. A closed circle (•) in Fig. 8 shows CRSP of the present invention, an open circle (○) shows pig calcitonin (pig CT) and an open rhombus (◇) shows salmon calcitonin (salmon CT).

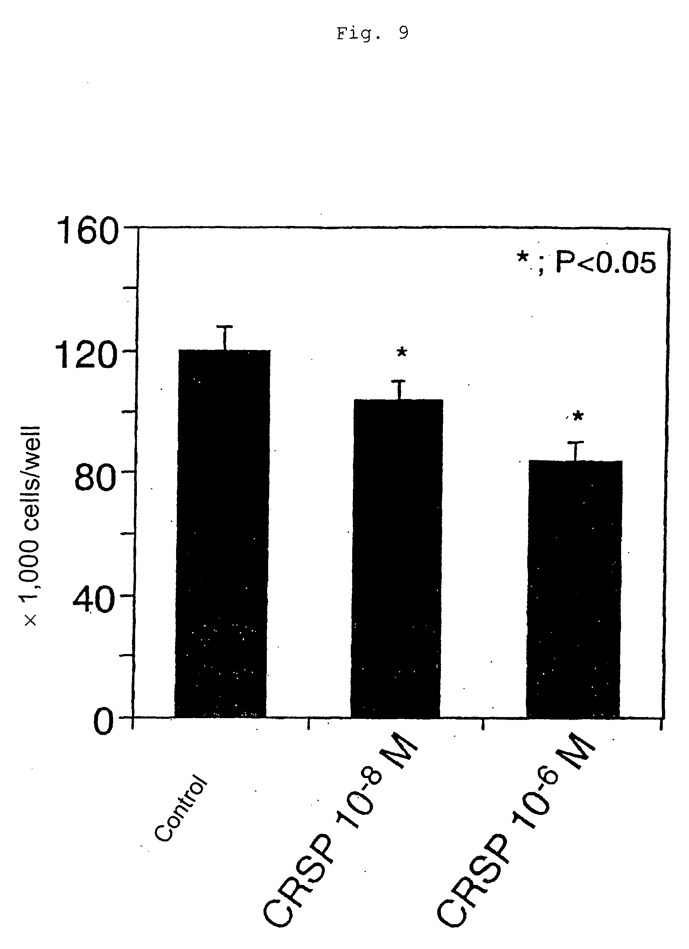

Fig. 9 shows the cells numbers resulting from cell proliferation by CRSP of the present invention in LLC-PK₁ cells measured by using a counting plate. The vertical axis in Fig. 9 shows cells numbers (× 1,000 cells/well), while the left end of the horizontal axis shows control (no CRSP added) and to the right thereof shows CRSP of each concentration. The mark * shows that there was a significant difference when p < 0.05.

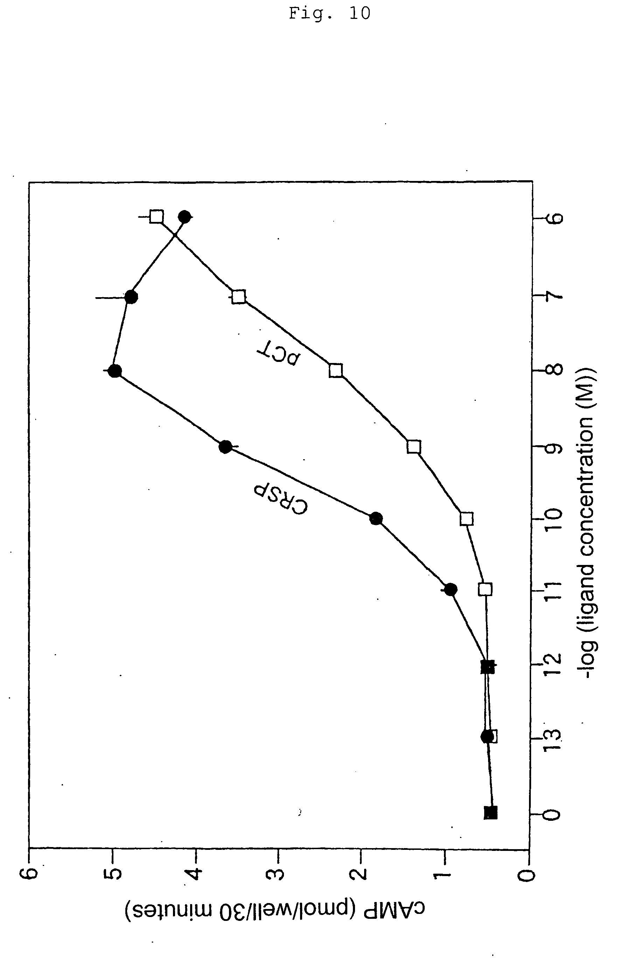

Fig. 10 shows the result of cAMP productivity measurement by CRSP stimulation of the peptide of the present invention in cells (COS-7) where calcitonin receptors are expressed in a genetic engineering manner. The vertical axis in Fig. 10 shows a production amount of CAMP (pmol/well/30 minutes) while the horizontal axis shows a reverse logarithm (-log (ligand concentration (M)) of concentration of each peptide (ligand). A closed circle (•) of Fig. 10 shows CRSP of the present invention and an open rhombus (◇) shows pig calcitonin (pCT).

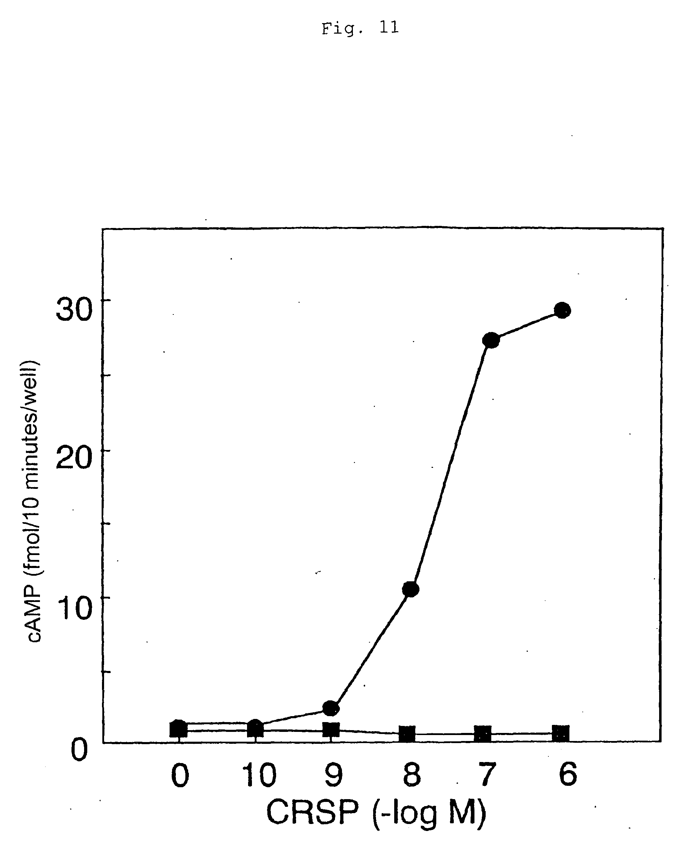

Fig. 11 shows the result of cAMP productivity measurement by CRSP stimulation of the peptide of the pres.ent invention in renal epithelial cells of opossum where pig calcitonin receptor (CTR) is expressed. The vertical axis in Fig. 11 shows production amount of cAMP (fmol/well/hour) while the horizontal axis shows a reverse logarithm (-log (CRSP concentration (M))) of concentration of CRSP. A closed circle (•) of Fig. 11 shows the case of opossum renal epithelial cells into which pig calcitonin receptor (CTR) is introduced and a closed square (■) shows the case of opossum renal epithelial cells (OK cells).

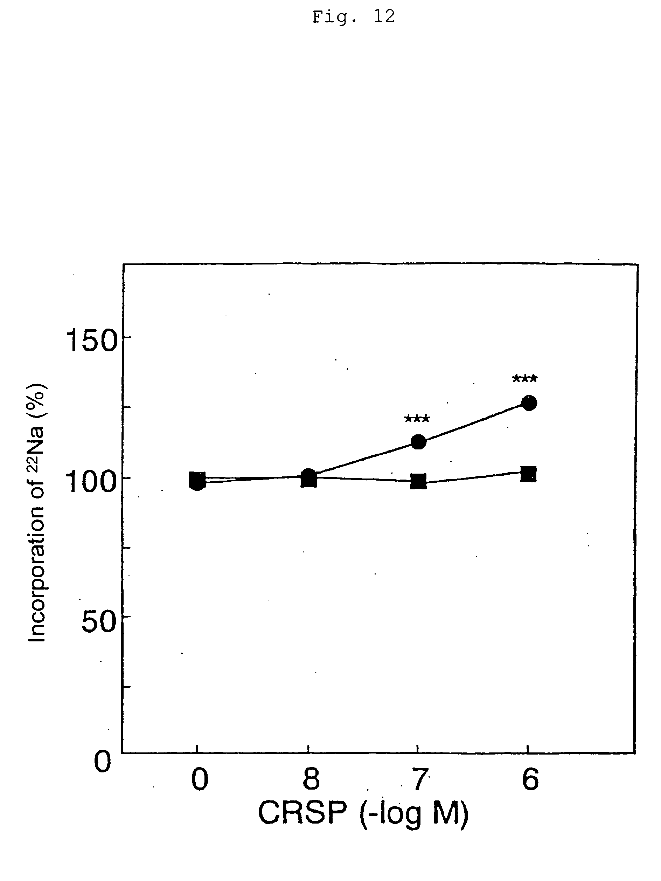

Fig. 12 shows the result of incorporation test of sodium ion by CRSP stimulation of the peptide of the present invention in opossum renal epithelial cells in which pig calcitonin receptors (CTR) are expressed. The vertical axis in Fig. 12 shows the ratio of incorporated amounts of sodium ion (where the control being 100) while the horizontal axis shows a reverse logarithm (-log (CRSP concentration (M))) of CRSP concentration. A closed circle (•) of Fig. 12 shows the case where opossum renal epithelial cells into which pig calcitonin receptor (CTR) is introduced and a closed square (■) shows the case of opossum renal epithelial cells (OK cells). The mark *** in Fig. 12 shows that there was a significant difference when p < 0.001.

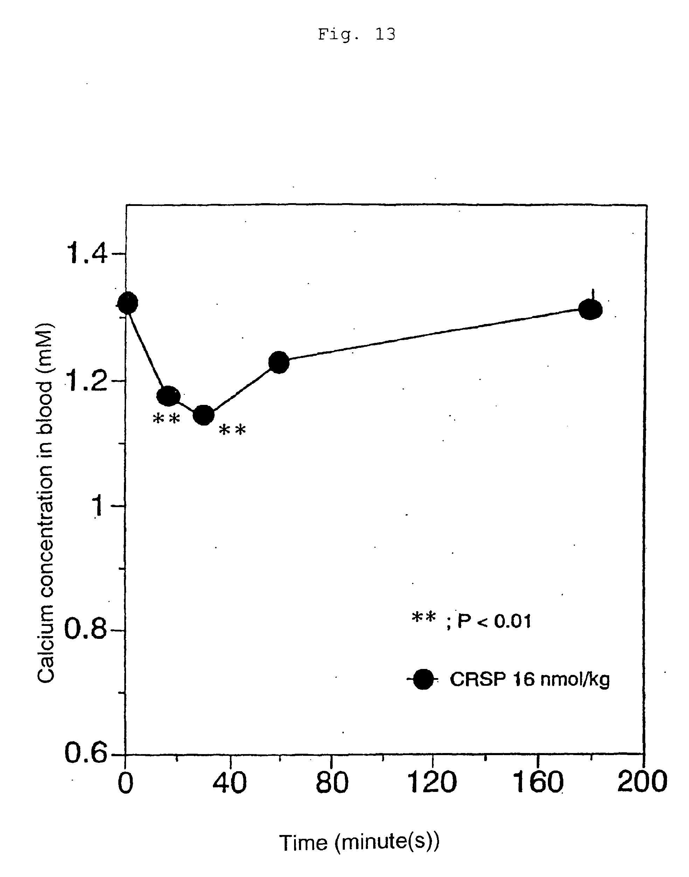

Fig. 13 shows the result of measurement of changes in calcium concentration in blood by administration of CRSP of the present invention in rats. The vertical axis in Fig. 13 shows a calcium concentration in blood (mM) while the horizontal axis shows time (minutes). The mark ** in Fig. 13 shows that there was a significant difference when p < 0.01.

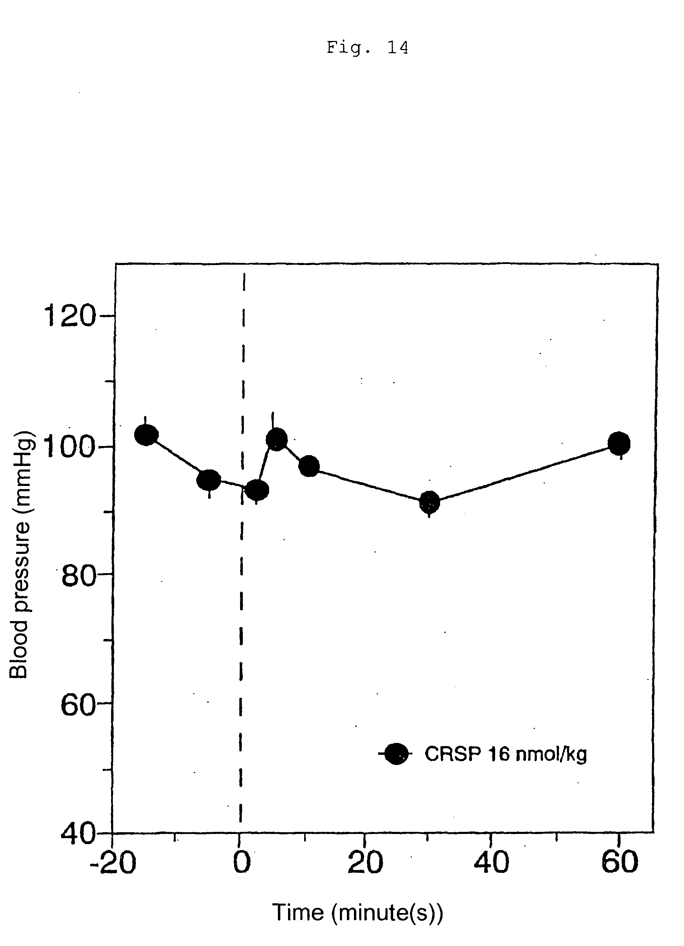

Fig. 14 shows the result of measurement of changes in blood pressure by administration of CRSP of the present invention in rats. The vertical axis in Fig. 14 shows blood pressure (mmHg) while the horizontal axis shows time (minutes).

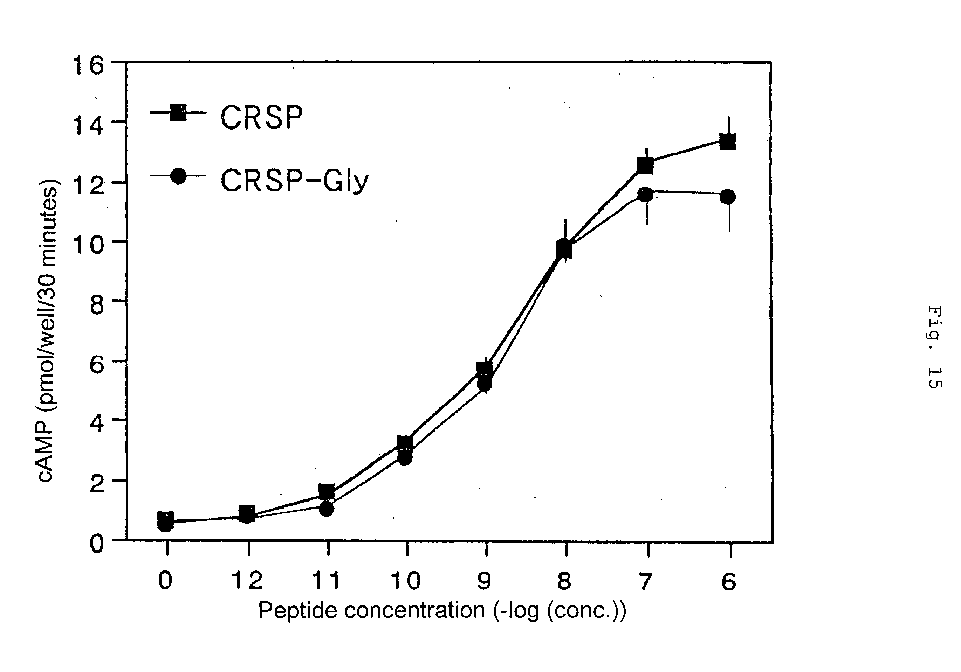

Fig. 15 shows the result of measurement by radioimmunoassay of cAMP production amount by CRSP or CRSP-Gly stimulation to cells where pig calcitonin receptor (CTR) is introduced into mammalian expression vector pcDNA 3.1 and expressed in renal cells of rhesus monkey (COS-7). The vertical axis shows production amount of cAMP (pmol/well/130 minutes) while the horizontal axis shows a reverse logarithm (-log (CRSP concentration (M))) of CRSP concentration.

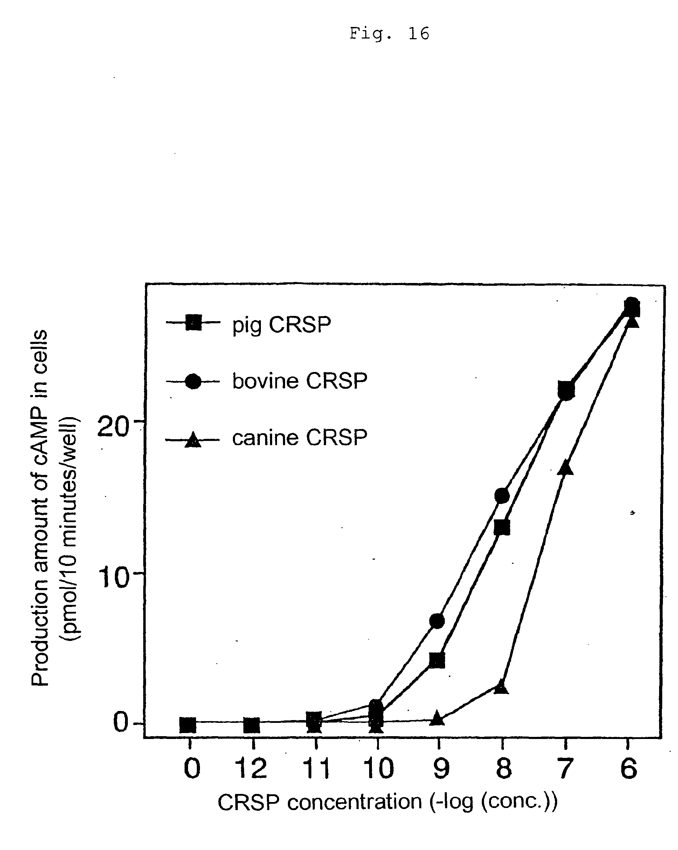

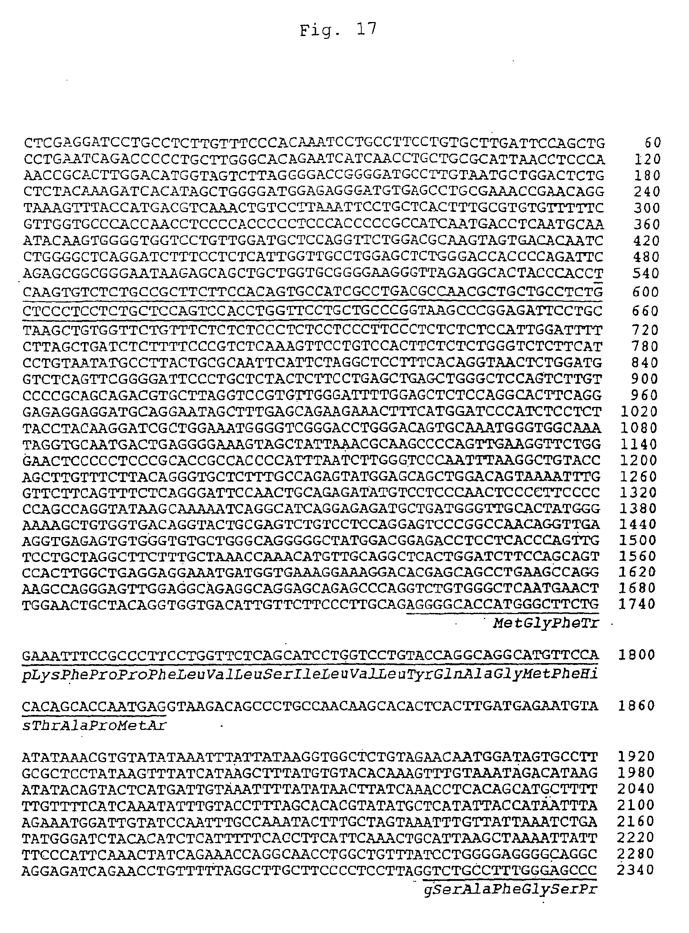

Fig. 16 shows the result of investigation of cAMP production-promoting activity of pig CRSP, bovine CRSP and canine CRSP by the peptide of the present invention using LLC-PK₁ cells. The vertical axis shows the production amount of cAMP (pmol/well/10 minutes) while the horizontal axis shows a reverse logarithm (-log (CRSP concentration (M))) of CRSP. In Fig. 16, (■) shows pig CRSP, (•) shows bovine CRSP and (▲) shows canine CRSP.

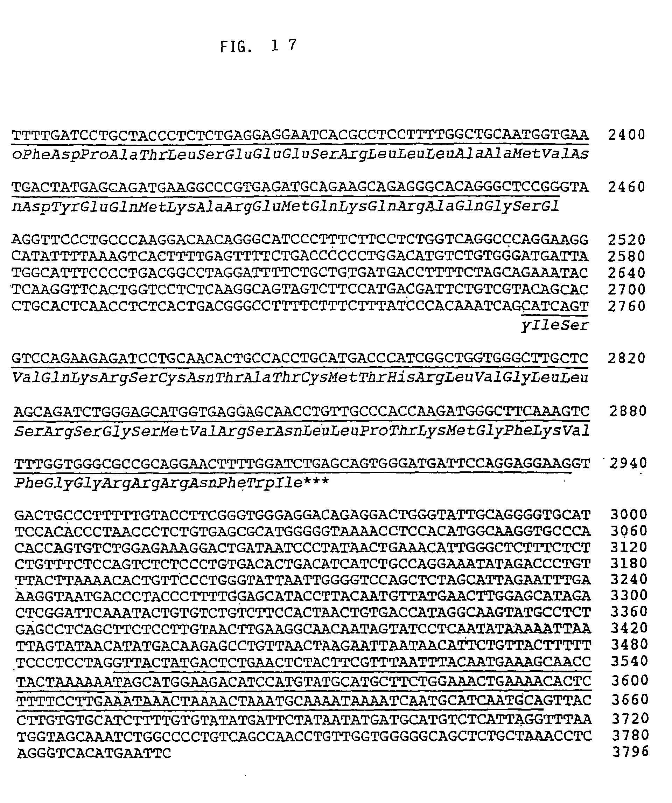

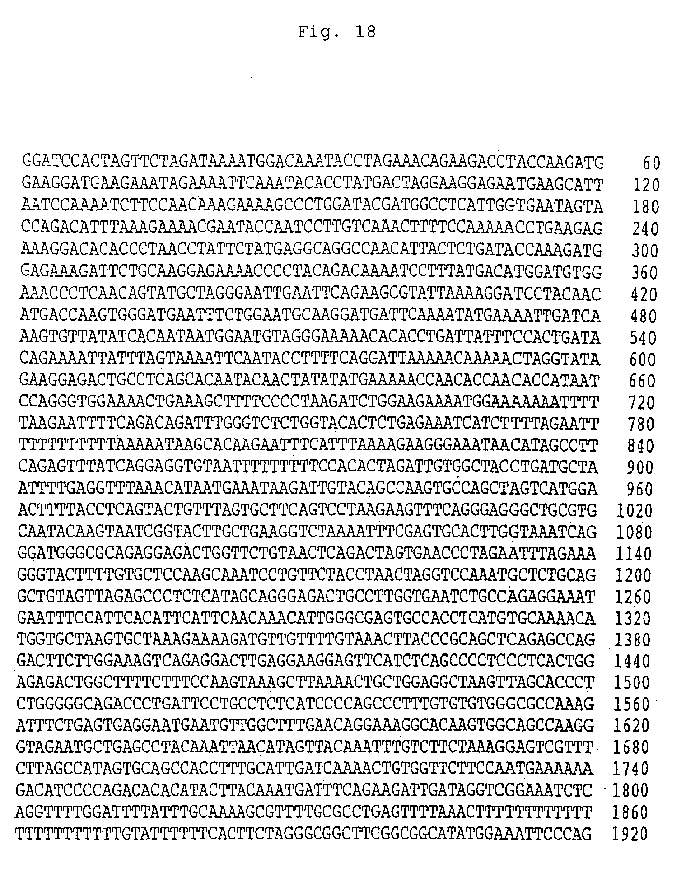

Fig. 17 shows a nucleotide sequence of CRSP gene. The underlined parts show exons. Beneath the underlined parts, amino acid sequences coded by CRSP gene are shown.

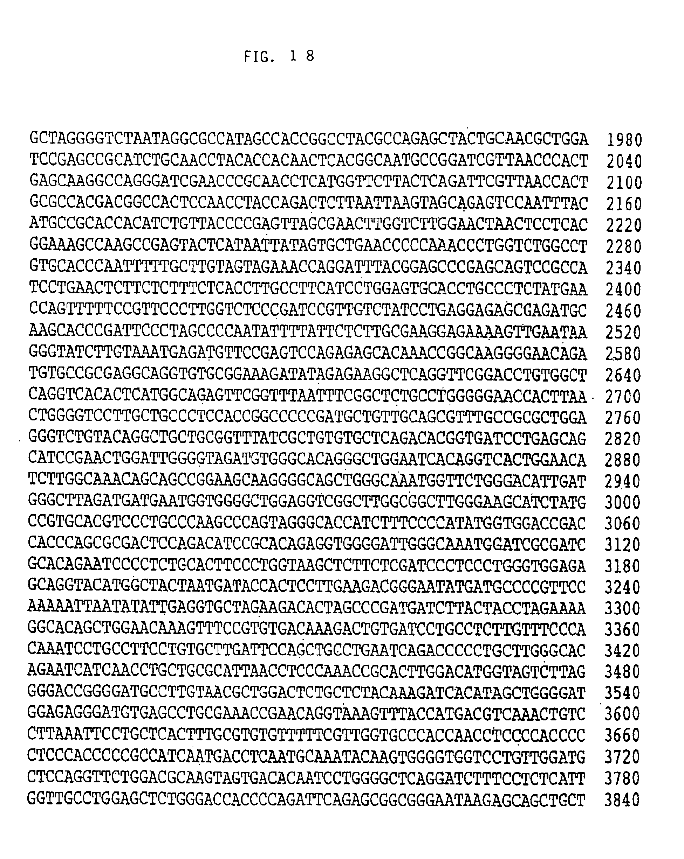

Fig. 18 shows the first half part (bases of from 1 to 3840) of the nucleotide sequence of CRSP-2 gene.

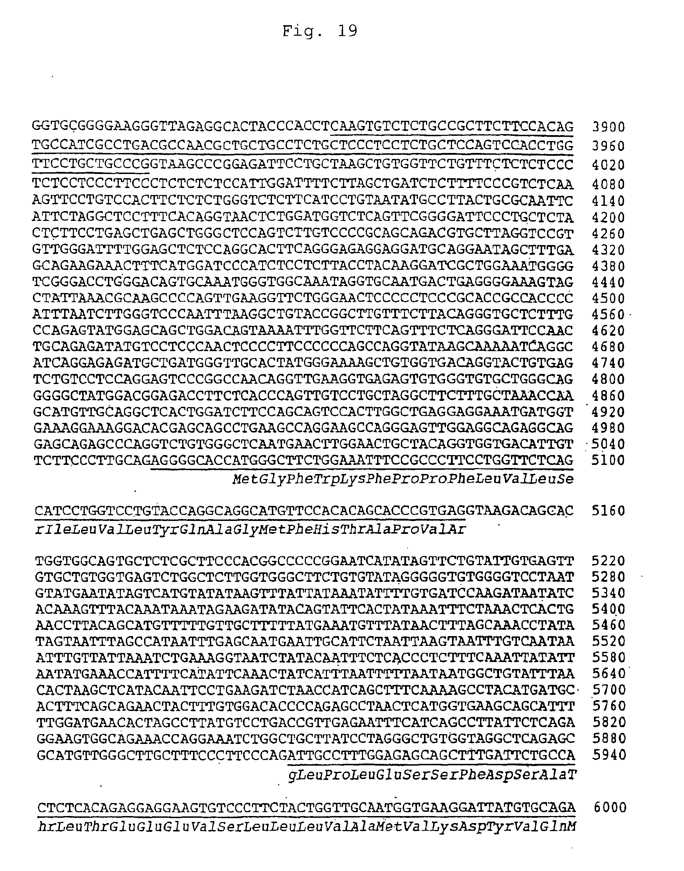

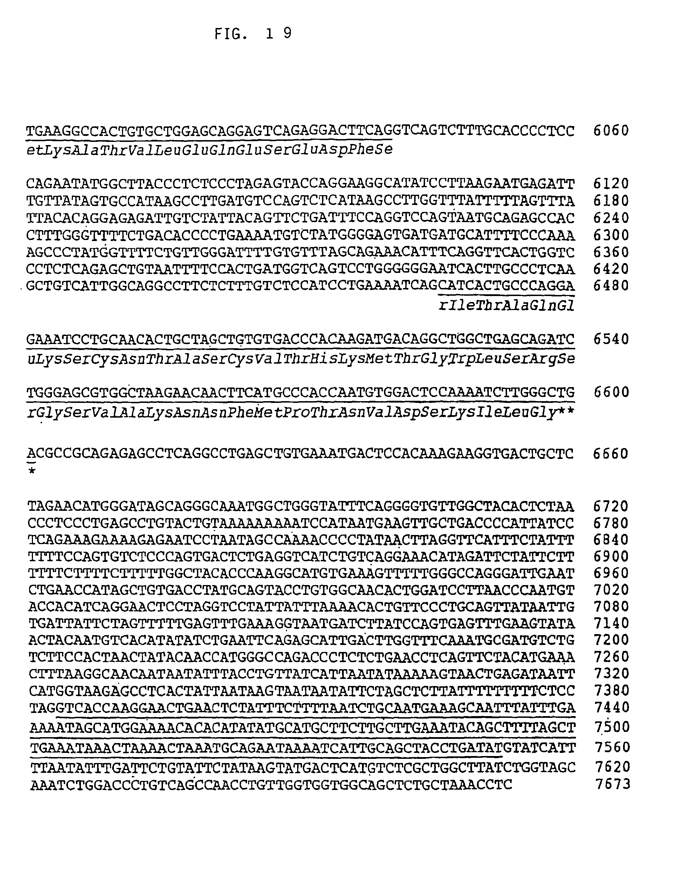

Fig. 19 shows the second half part (bases of from 3841 to 7673) of the nucleotide sequence of CRSP-2 gene. The underlined parts show exons. Beneath the underlined parts, amino acid sequences coded by CRSP-2 gene are shown.

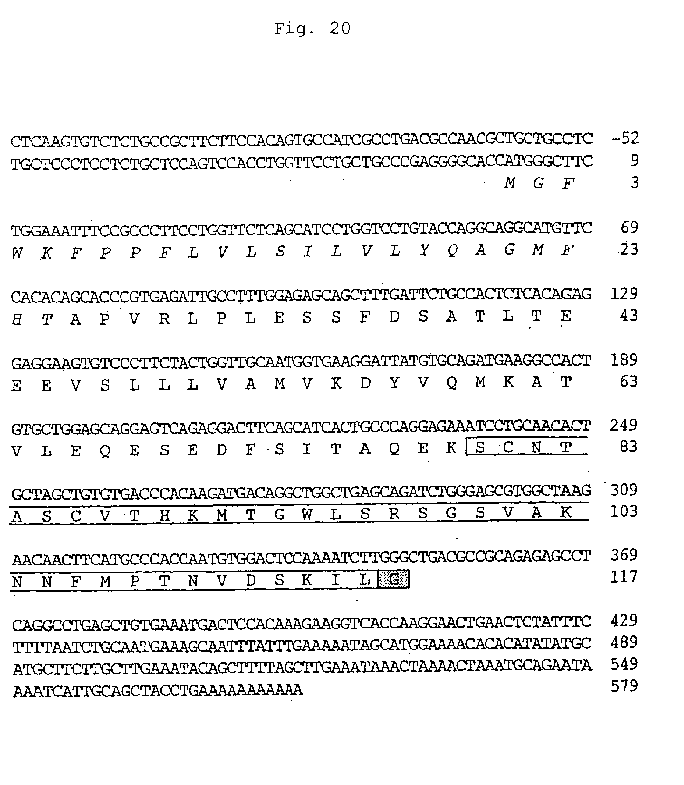

Fig. 20 shows a cDNA nucleotide sequence of CRSP-2. In Fig. 20, an area enclosed by a solid line is a mature peptide and the part in gray color shows glycine which is presumed to be converted to an amide.

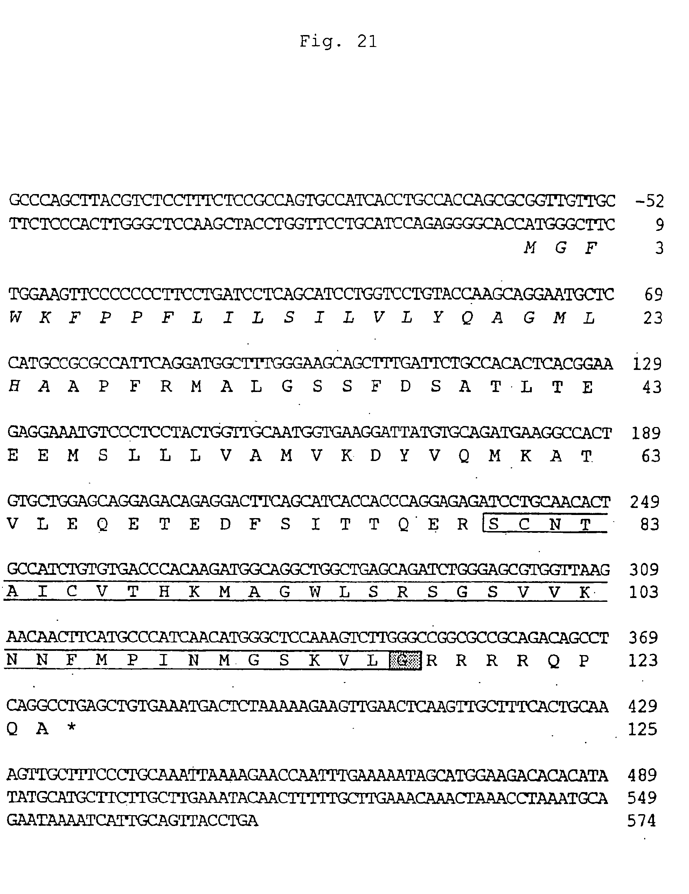

Fig. 21 shows a cDNA nucleotide sequence of CRSP-3. In Fig. 21, the area enclosed by a solid line is a mature peptide and the part in gray color shows glycine which is presumed to be converted to an amide.

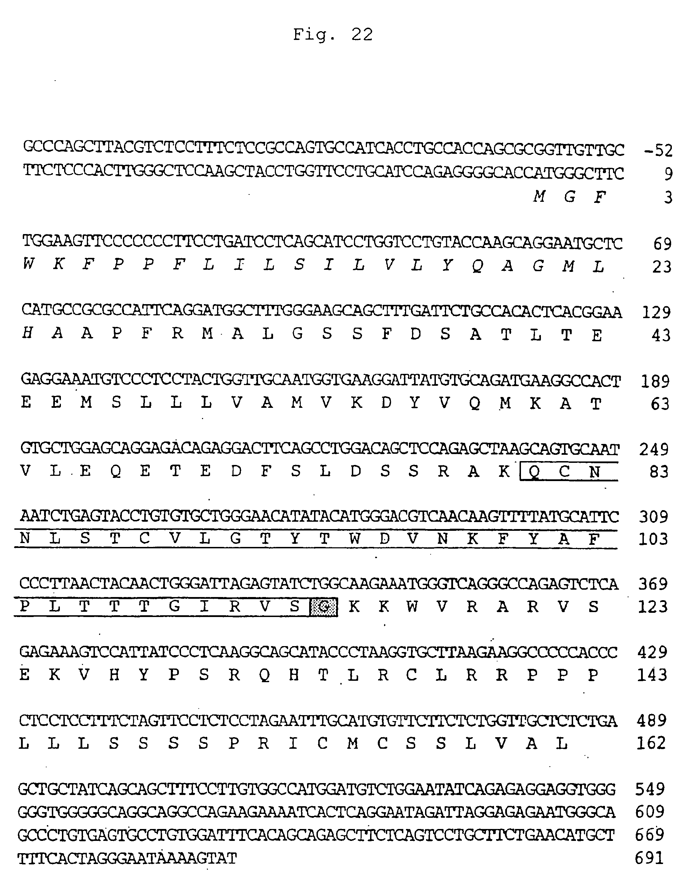

Fig. 22 shows a DNA nucleotide sequence of CT-2. In Fig. 22, an area enclosed by a solid line is a mature peptide and the part in gray color shows glycine which is presumed to be converted to an amide. Glutamine located at the N-terminal side of mature substance in Fig. 22 is presumed to be converted to pyroglutamic acid.

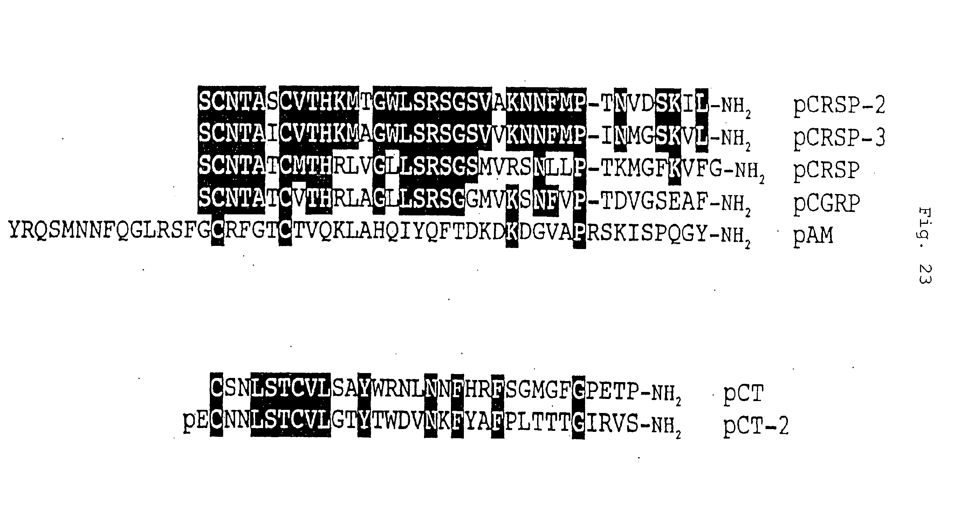

Fig. 23 shows a drawing where amino acids of CRSP, CGRP, AM, CT-2 and CT are compared.

There is shown comparison of amino acid sequences of CRSP (pCRSP), CRSP-2 (pCRSP-2), CRSP-3 (pCRSP-3) and CT-2 (pCT-2) which are peptides of the present invention with amino acid sequences of pig calcitonin gene-related peptide (pCGRP), pig calcitonin (pCT) and pig adrenomedullin (pAM).

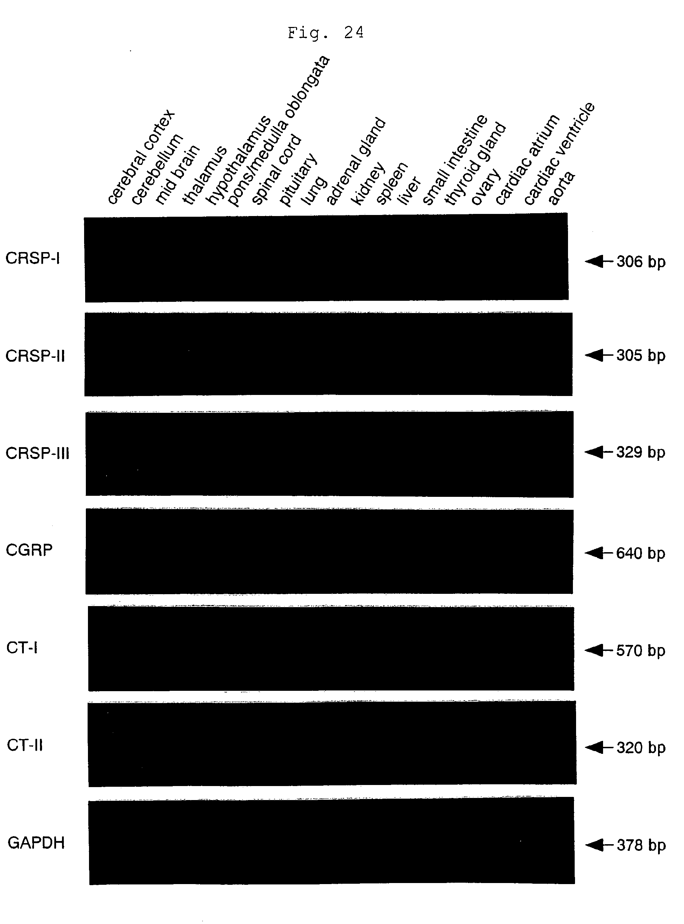

Fig. 24 shows the result of a high-sensitivity quantitative determination, by RT-PCR, of expressed amount of gene of CRSP, CRSP-2, CRSP-3, CT-2, CT and CGRP. The vertical axis in the drawing shows various genes while the horizontal axis shows tissues of pigs where expressed amount of gene was measured. In order to normalize total amount of RNA, expressed amount of GAPDH (glyceraldehydes-3-phosphate dehydrogenase) was also measured.

### Best Mode for Carrying Out the Invention

The present inventors have purified two kinds of novel physiologically active peptides from an extract of porcine brain using a cAMP production of renal epithelial cells as an index. When the amino acid sequence of the resulting peptides is analyzed, it has been found that they are a peptide (hereinafter, it will be referred to as CRSP) comprising the following 38 amino acids Ser-Cys-Asn-Thr-Ala-Thr-Cys-Met-Thr-His-          10 Arg-Leu-Val-Gly-Leu-Leu-Ser-Arg-Ser-Gly-        20 Ser-Met-Val-Arg-Ser-Asn-Leu-Leu-Pro-Thr-        30 Lys-Met-Gly-Phe-Lys-Val-Phe-Gly-NH2 38 and a peptide (hereinafter, it will be referred to as CRSP-G) comprising the following 39 amino acids where glycine is bonded to a C-terminal of the above described peptide.

Ser-Cys-Asn-Thr-Ala-Thr-Cys-Met-Thr-His-          10

Arg-Leu-Val-Gly-Leu-Leu-Ser-Arg-Ser-Gly-        20

Ser-Met-Val-Arg-Ser-Asn-Leu-Leu-Pro-Thr-          30

Lys-Met-Gly-Phe-Lys-Val-Phe-Gly-Gly-OH 39

When the amino acid sequences of those peptides were searched by databases (Genbank, swissprot, DDBJ), both have been found to be confirmed to have novel amino acid sequences.

Those amino acid sequences are shown in SEQ NO: 1 and SEQ ID NO: 2 of the Sequence Listing, respectively.

A synthetic primer was prepared on the basis of the amino acid sequence of the resulting peptide and amplified by a PCR using pig gene as a template whereupon an aimed gene was cloned.

On the basis of the amino acid sequence, the primer used was 
TG(C/T)AA(C/T)AC(A/C/G/T)GC(A/C/G/T)AC(A/C/G/T)TG(C/
T)ATGAC
 at the N-terminal side and was CC(A/G)AA(A/C/G/T)AC(C/T)TT(A/G)AA(A/C/G/T)CCCATA at the C-terminal side.

A base sequence of the resulting gene is shown in SEQ ID NO: 3 of Sequence Listing and an amino acid sequence coded thereby is shown as follows. Met-Gly-Phe-Trp-Lys-Phe-Pro-Pro-Phe-Leu-          10 Val-Leu-Ser-Ile-Leu-Val-Leu-Tyr-Gln-Ala-        20 Gly-Met-Phe-His-Thr-Ala-Pro-Met-Arg-Ser-          30 Ala-Phe-Gly-Ser-Pro-Phe-Asp-Pro-Ala-Thr- 40 Leu-Ser-Glu-Glu-Glu-Ser-Arg-Leu-Leu-Leu-          50 Ala-Ala-Met-Val-Asn-Asp-Tyr-Glu-Gln-Met-          60 Lys-Ala-Arg-Glu-Met-Gln-Lys-Gln-Arg-Ala-          70 Gln-Gly-Ser-Gly-Ile-Ser-Val-Gln-Lys-Arg-          80 Ser-Cys-Asn-Thr-Ala-Thr-Cys-Met-Thr-His-          90 Arg-Leu-Val-Gly-Leu-Leu-Ser-Arg-Ser-Gly- 100 Ser-Met-Val-Arg-Ser-Asn-Leu-Leu-Pro-Thr-          110 Lys-Met-Gly-Phe-Lys-Val-Phe-Gly-Gly-Arg-          120 Arg-Arg-Asn-Phe-Trp-Ile 126 (In the formula, the underlined 81st to 118th ones are CRSP.)

The amino acid sequence is shown in SEQ ID NO: 4 of Sequence Listing.

Using pig CRSP cDNA as a probe, the peptide of the present invention was prepared from a library of bovine and canine thyroid cDNA.

An amino acid sequence of bovine CRSP represented by one-letter codes for amino acids is as follows.

ACNTATCMTHRLAGWLSRSG

SMVRSNLLPTKMGFKIFNGP-OH

When it is represented by three-letter codes for amino acids, it is as follows.

Ala-Cys-Asn-Thr-Ala-Thr-Cys-Met-Thr-His-          10

Arg-Leu-Ala-Gly-Trp-Leu-Ser-Arg-Ser-Gly-          20

Ser-Met-Val-Arg-Ser-Asn-Leu-Leu-Pro-Thr-          30

Lys-Met-Gly-Phe-Lys-Ile-Phe-Asn-Gly-Pro-OH 40

An amino acid sequence of canine CRSP represented by one-letter codes for amino acids is as follows.

SCNSATCVAHWLGGLLSRAG

SVANTNLLPTSMGFKVYN-OH

When it is represented by three-letter codes for amino acids, it is as follows.

Ser-Cys-Asn-Ser-Ala-Thr-Cys-Val-Ala-His-        10

Trp-Leu-Gly-Gly-Leu-Leu-Ser-Arg-Ala-Gly-        20

Ser-Val-Ala-Asn-Thr-Asn-Leu-Leu-Pro-Thr-          30

Ser-Met-Gly-Phe-Lys-Val-Tyr-Asn-OH 38

Each of those amino acid sequences, nucleotide sequences and total amino acid sequences coded by gene is shown in Sequence Listing.

An amino acid sequence of the peptide of the present invention of bovine is shown in SEQ ID NO: 6 of Sequence Listing, a nucleotide sequence of the gene thereof is shown in SEQ ID NO: 7 of Sequence Listing and an amino acid sequence of a peptide on the basis of the gene is shown in SEQ ID NO: 8.

An amino acid sequence of the peptide of the present invention of canine is shown in SEQ ID NO: 9 of Sequence Listing, a nucleotide sequence of the gene thereof is shown in SEQ ID NO: 10 of Sequence Listing and an amino acid sequence of a peptide on the basis of the gene is shown in SEQ ID NO: 11.

Using the full length of coding region of pig CRSP cDNA as a probe, gene coding for a peptide having a homology to CRSP was prepared from a pig genomic library. Peptides which are products of the gene are named CRSP-2 and CRSP-3, respectively.

Similarly, gene coding for a peptide having a homology to pig calcitonin (CT) was identified. A peptide which is a product of the gene was named CT-2.

An amino acid sequence of CRSP-2 as represented by three-letter codes for amino acids is as follows.

Ser-Cys-Asn-Thr-Ala-Ser-Cys-Val-Thr-His-          10

Lys-Met-Thr-Gly-Trp-Leu-Ser-Arg-Ser-Gly-          20

Ser-Val-Ala-Lys-Asn-Asn-Phe-Met-Pro-Thr-          30

Asn-Val-Asp-Ser-Lys-Ile-Leu-NH2 37

An amino acid sequence of CRSP-3 as represented by three-letter codes for amino acids is as follows.

Ser-Cys-Asn-Thr-Ala-Ile-Cys-Val-Thr-His-     10

Lys-Met-Ala-Gly-Trp-Leu-Ser-Arg-Ser-Gly-     20

Ser-Val-Val-Lys-Asn-Asn-Phe-Met-Pro-Ile-     30

Asn-Met-Gly-Ser-Lys-Val-Leu-NH2 37

An amino acid sequence of CT-2 as represented by three-letter codes for amino acids is as follows.

Glu-Cys-Asn-Asn-Leu-Ser-Thr-Cys-Val-Leu-     10

Gly-Thr-Tyr-Thr-Trp-Asp-Val-Asn-Lye-Phe-          20

Tyr-Ala-Phe-Pro-Leu-Thr-Thr-Thr-Gly-Ile-        30

Arg-Val-Ser-NH2 33

An amino acid sequence of CRSP-2 is shown in SEQ ID NO: 12 of Sequence Listing, a nucleotide sequence of cDNA thereof is shown in SEQ ID NO: 13 and an amino acid sequence of a peptide on the basis of the cDNA (a precursor peptide) is shown in SEQ ID NO: 14. A nucleotide sequence of CRSP-2 gene is shown in SEQ ID NO: 15.

An amino acid sequence of CRSP-3 is shown in SEQ ID NO: 16 of Sequence Listing, a nucleotide sequence of cDNA thereof is shown in SEQ ID NO: 17 and an amino acid sequence of a peptide on the basis of the cDNA (a precursor peptide) is shown in SEQ ID NO: 18.

An amino acid sequence of CT-2 is shown in SEQ ID NO: 19 of Sequence Listing, a nucleotide sequence of cDNA thereof is shown in SEQ ID NO: 20 and an amino acid sequence of a peptide on the basis of the cDNA (a precursor peptide) is shown in SEQ ID NO: 21. A nucleotide sequence of CRSP-3 gene and CT-2 gene is shown in SEQ ID NO: 22.

In Fig. 1, a structure of pig CRSP is schematically shown as an example of the peptides of the present invention. In the case of the peptide of this example, the second Cys and the seventh Cys are in an -S-S- bond mode.

Fig. 2 shows comparison of amino acid sequences of pig CRSP (pCRSP) of the peptide of the present invention, pig calcitonin gene-related peptide (pCGRP-I), human calcitonin gene-related peptide I (hCGRP-I), human calcitonin gene-related peptide II (hCGRP-II), human amylin (hAmylin), pig calcitonin (pCT) and human adrenomedullin (hAM).

The CRSP of the present invention has a homology in terms of amino acid sequence to pig calcitonin gene-related peptide (pCGRP), human CGRP-I and human CGRP-II to an extent of 71.1%, 63.2% and 71.1%, respectively.

Comparison of amino acids for CRSP, CRGP, AM, CT-2 and CT with regard to peptides derived from pig is shown in Fig. 23.

The result of Northern blotting for investigating the expression site of CRSP is shown in Fig. 3 by a photograph as a drawing. An arrow in Fig. 3 shows the position of CRSP mRNA. Further, content of peptides in each tissue was measured by means of radioimmunoassay using antibody against CRSP. Result of the measurement is shown in the following Table 1.

**Table 1**

| Concentration of CRSP in Tissues as Measured by Radioimmunoassay | |
|---|---|
| Tissues | Immunoactivity of CRSP (pmol/g tissue) |
| Cerebral cortex | 0.29 ± 0.04 |
| Cerebellum | 0.18 ± 0.02 |
| Midbrain | 7.5 ± 0.9 |
| Hippocampus | 0.78 ± 0.16 |
| Caudatum | 1.3 ± 0.1 |
| Thalamus | 3.5 ± 0.4 |
| Hypothalamus | 9.9 ± 1.2 |
| Pons/Medulla oblongata | 2.2 ± 0.3 |
| Spinal cord | 0.52 ± 0.06 |
| Olfactory bulb | 0.74 ± 0.22 |
| Anterior pituitary | 14 ± 2 |
| Posterior pituitary | 96 ± 15 |
| Lung | 0.11 ± 0.00 |
| Adrenal gland | 0.42 ± 0.05 |
| Kidney/Cortex | 0.12 ± 0.01 |
| Kidney/Medulla | 0.088 ± 0.039 |
| Liver | 0.13 ± 0.02 |
| Spleen | 0.11 ± 0.01 |
| Stomach | 0.29 ± 0.00 |
| Small intestine | 0.072 ± 0.018 |
| Pancreas | 0.066 ± 0.010 |
| Thyroid gland | 68 ± 39 |
| Ovary | 0.18 ± 0.09 |
| Cardiac atrium | 0.20 ± 0.04 |
| Cardiac ventricle | 0.21 ± 0.09 |
| Aorta | 0.33 ± 0.19 |
| Each value represents mean ± standard error (n = 3) | |

As a result, high levels of gene expression regarding CRSP of the present invention have been detected in central nervous system such as midbrain and hypothalamus, and in peripheral system such as thyroid gland. Moderate expression was detected in pons/medulla oblongata. Expression, though only a little, was detected even in cerebrum and pituitaries. On the other hand, the concentration of CRSP in tissues was most abundant in posterior pituitary and thyroid gland, and also high in midbrain, hypothalamus and anterior pituitary.

Biological activity of CRSP was then investigated.

Firstly, cAMP productivity of CRSP was investigated using LLC-PK₁ cells.

CRSP dissolved in DMEM was added to a medium for LLC-PK₁ cells and amount of cAMP secreted therefrom was quantified by means of radioimmunoassay using a cAMP-specific antibody. The result is shown in Fig. 4. The vertical axis in Fig. 4 shows the production amount of cAMP (pmol/10⁵ cells/30 minutes) while the horizontal axis thereof shows a reverse logarithm (-log (peptide concentration(M))) of concentration of each peptide. A closed circle (•) of Fig. 4 shows CRSP of the present invention, an open circle (○) shows pig calcitonin gene-related peptide (pig CGRP) and an open rhombus (◇) shows pig calcitonin (pig CT). As a result, CRSP of the present invention very strongly increases (ED₅₀ being about 1.5 nM) an intracellular adenylyl cyclase activity of LLC-PK₁ cells derived from pig renal epithelial cells in a concentration-dependent manner. The activity was stronger than pig calcitonin (ED₅₀ being about 8. 7 nM) and pig CGRP (ED₅₀ being about 62 nM) to an extent of about 6-fold and about 40-fold, respectively.

Then, CRSP of the present invention was investigated in function of promoting incorporation of sodium ion and calcium ion.

A culture solution of LLC-PK₁ cells was substituted with a Hanks' choline chloride solution, CRSP and sodium chloride having a predetermined radioactivity (²²Na) were added thereto so as to make the final concentration 0 M, 10⁻⁸ M, 10⁻⁷ M and 10⁻⁶ M each, the cells were washed after 10 minutes so that ²²Na contained in the solution outside the cells was completely removed and radioactivity incorporated into the cells was measured by a gamma-counter. Similarly, a culture solution of LLC-PK₁ cells was substituted with a Hanks' choline chloride solution, sodium chloride having a predetermined radioactivity (²²Na) as well as CRSP and an amyloride derivative (5-(N-ethyl-N-isopropyl)-amiloride) (EIPA) which is an Na/H co-transporter inhibitor were added thereto so as to make the final concentration 0 M CRSP + 0 M EIPA, 10⁻⁶ M CRSP + 0 M EIPA, 10⁻⁶ M CRSP + 10⁻⁸ M EIPA, 10⁻⁶ M CRSP + 10⁻⁷ M EIPA, 10⁻⁶ M CRSP + 10⁻⁶ M EIPA and 0 M CRSP + 10⁻⁶ M EIPA each. Cells were washed after 10 minutes so that ²²Na contained in the outer solution of the cells was completely removed and radioactivity incorporated into the cells was measured by a gamma-counter.

The results are shown in Fig. 5 (incorporation of sodium ion in the absence of EIPA), Fig. 6 (incorporation of sodium ion in the presence of EIPA) and Fig. 7 (incorporation of calcium ion). The vertical axes in Fig. 5, Fig. 6 and Fig. 7 show incorporated amounts (cpm) of each ion, while the left end of the horizontal axis shows control (no peptide added) and to the right thereof shows CRSP for each concentration. Right side of the control in the horizontal axis in Fig. 6 shows the case of a sole administration of CRSP, the right side thereof shows each concentration of co-administration of CRSP with EIPA and the right end shows the case of a sole administration of EIPA. sCT in Fig. 7 shows salmon calcitonin. In Fig. 5, Fig. 6 and Fig. 7, the marks *, ** and *** show that there was a significant different when p < 0.05, p < 0.01 and p < 0.001, respectively.

As a result, it was found that CRSP activated an amyloride-sensitive Na/H co-transporter on LLC-PK₁ cells whereby incorporation of sodium into cells was promoted and incorporation of calcium into cells was suppressed.

A suppressive action of CRSP for cell proliferation was then investigated.

A solution of each peptide in DMEM in each concentration was added to LLC-PK₁ cells, then DMEM (containing 0.1% BSA) which contained bromodeoxyuridine labeled with ¹²⁵I (¹²⁵I-DU) was added to each well and, after 5 hours, radioactivity incorporated into the nuclei was measured by a gamma-counter to measure the DNA amount synthesized as a result of cell proliferation. The result is shown in Fig. 8. The vertical axis in Fig. 8 shows incorporated amount of ¹²⁵I-DU (× 100 cpm/well) while the horizontal axis shows a reverse logarithm (-log (peptide concentration (M))) of each peptide. A closed circle (•) in Fig. 8 shows CRSP of the present invention, an open circle (○) shows pig calcitonin (pig CT) and an open rhombus (◇) shows salmon calcitonin (salmon CT).

With regard to the number of proliferated cells, each solution of CRSP in DMEM in various concentrations was added to LLC- PK₁ cells of 10,000 cells/well, incubation was conducted, the cells on the incubation dish was taken out by a trypsin-EDTA solution and cell numbers were counted using a counting plate. The result is shown in Fig. 9. The vertical axis in Fig. 9 shows cell numbers (× 1,000 cells/well), while the left end of the horizontal axis shows control (no CRSP added) and to the right thereof shows CRSP of each concentration. The mark * shows that there was a significant difference when p < 0.05.

As a result thereof, it was found that CRSP has an action of suppressing the growth of LLC-PK₁ cells.

Action of CRSP of the present invention on calcitonin receptor was investigated.

Pig calcitonin receptor (CTR) was introduced into a mammalian expression vector pcDNA 3.1, expressed in rhesus monkey renal cells (COS-7) and production amount of cAMP by stimulation of CRSP was measured by radioimmunoassay. Similarly, CTR was expressed in opossum renal cells (OK cells) and production amount of cAMP by stimulation of CRSP was measured by radioimmunoassay while incorporation of sodium ion was measured by measuring the incorporation of sodium chloride having radioactivity (²²Na) by a gamma-counter. The results are shown in Fig. 10 (changes in cAMP productivity by CRSP stimulation in cells (COS-7) where calcitonin receptor was expressed in a genetic engineering manner), Fig. 11 (changes in cAMP productivity in opossum renal epithelial cells where pig calcitonin receptor (CTR) was expressed) and Fig. 12 (changes in incorporation of sodium ion in opossum renal epithelial cells where pig calcitonin receptor (CTR) was expressed).

The vertical axis in Fig. 10 shows a production amount of cAMP (pmol/well/30 minutes) while the horizontal axis shows a reverse logarithm (-log (ligand concentration (M))) of concentration of each peptide (ligand). A closed circle (•) of Fig. 10 shows CRSP of the present invention and an open rhombus (◇) shows pig calcitonin (pCT). The vertical axis in Fig. 11 shows production amount of cAMP (fmol/well/hour) while the horizontal axis shows a reverse logarithm (-log (CRSP concentration (M))) of concentration of CRSP. A closed circle (•) of Fig. 11 shows the case of opossum renal epithelial cells into which pig calcitonin receptor (CTR) is introduced and a closed square (■) shows the case of opossum renal epithelial cells (OK cells). The vertical axis in Fig. 12 shows the ratio of incorporated amounts of sodium ion (where the control being 100) while the horizontal axis shows a reverse logarithm (-log (CRSP concentration (M))) of CRSP concentration. A closed circle (•) of Fig. 12 shows the case where opossum renal epithelial cells into which pig calcitonin receptor (CTR) is introduced and a closed square (■) shows the case of opossum renal epithelial cells (OK cells). A mark *** in Fig. 12 shows that there was a significant difference when p < 0.001.

As a result, it was noted that, when CTR was expressed in COS-7 and stimulated by CRSP, intracellular adenylyl cyclase activity increases (ED₅₀ being about 0.2 nM). The activity was stronger than pig calcitonin (ED₅₀ being about 71 nM) to an extent of about 350-fold. Such an action of CRSP was stronger than pig calcitonin to an extent of about 350-fold and was strongest among the known physiologically active peptides. It was similarly noted that, when CTR was expressed in opossum renal epithelial cells (OK cells) and stimulation was conducted by CRSP, intracellular adenylyl cyclase activity increases and, at the same time, an amyloride-sensitive Na/H co-transporter was activated whereupon incorporation of sodium into cells was promoted.

Changes in blood pressure and ion concentration in plasma in rats by CRSP were also investigated.

CRSP corresponding to an amount of 16 nmol/kg dissolved in a physiological saline solution was administered at a time within a short period from jugular vein of rat under an anesthetized condition and calcium concentration in the plasma and blood pressure were measured. The results are shown in Fig. 13 (changes in calcium concentration in blood by administration of CRSP) and Fig. 14 (changes in blood pressure by administration of CRSP). The vertical axis in Fig. 13 shows a calcium concentration in blood while the horizontal axis shows time (minutes). The vertical axis in Fig. 14 shows blood pressure (mmHg) while the horizontal axis shows time (minutes). A mark ** in Fig. 13 shows that there was a significant difference when p < 0.01.

As a result, it was noted that, when CRSP was administered, calcium concentration in plasma transiently lowered while there was no apparent change in blood pressure by that.

Then, the difference in cAMP production-promoting activities by CRSP and CRSP-Gly was investigated.

Pig calcitonin receptor (CTR) was introduced into mammalian expression vector pcDNA 3.1 and then expressed in rhesus monkey renal cells (COS-7) whereupon production amount of cAMP by CRSP stimulation was measured by radioimmunoassay. The result is shown in Fig. 15.

As a result, it was noted that CRSP and CRSP-Gly have nearly the same cAMP productivity.

Further, with regard to the peptides of the present invention identified in cow and dog (bovine CRSP and canine CRSP), cAMP productivity of CRSP using LLC-PK₁ cells by the same manner as above was investigated. The result is shown in Fig. 16. The vertical axis in Fig. 16 shows production amount of cAMP (pmol/well/10 minutes) while the horizontal axis shows the concentration of CRSP added thereto.

As a result, it was found that even bovine and canine CRSPs achieved nearly the same result as in the case of pig CRSP.

Expressed amounts of gene by various peptides of the present invention, CRSP, CRSP-2, CRSP-3, CT-2, CT, CGRP and GAPDH were measured by a high-sensitivity quantitative determination using an RT-PCR. The result is shown in Fig. 24.

With regard to CRSP-2 and CRSP-3, the expression was observed in the central nervous system, thyroid gland and ovary. On the contrary, with regard to CT-2, its band was not amplified in any tissue whereby no expression was observed. It is presumed that CT-2 is expressed only in other areas than the tissues shown in Fig. 24.

As described above, it is noted that the peptides of the present invention stimulate calcitonin receptor in a stronger manner than calcitonin whereupon adenylyl cyclase is activated by that. The above shows that the peptide of the present invention is a genuine endogenous ligand for calcitonin receptor.

Accordingly, the peptides of the present invention are believed to act on the calcitonin receptor in peripheral tissues and elicit the following pharmaceutical effects.

Calcitonin promotes incorporation of calcium into bone via calcitonin receptor which is a receptor of calcitonin. It is concluded that, like calcitonin, the peptides of the present invention act on calcitonin receptor and promote the incorporation of calcium into bone. Actually, as shown in Fig. 13, a decrease in calcium concentration in plasma was detected in rats by administration of the peptide. It is concluded that the result supports the promotion of incorporation of calcium into bones. By utilizing such a function, the peptides are applicable as a drug for recovery of bone density, reduced due to osteoporosis, to a healthy state and can be used as preventive and treating agents for osteoporosis.

In addition, as shown in Fig. 13, the present peptides have an activity of lowering the calcium concentration in plasma whereupon they are applicable as drugs for lowering the calcium concentration inplasma of hypercalcemia as a result of alkalosis, etc. to a normal level and can be used as treating and preventive agents for hypercalcemia.

The peptides of the present invention activate the action of an amyloride-sensitive Na/H co-transporter of renal epithelial cells. That is the activity opposed to amyloride which is a potassium-retaining diuretic agent, whereby they can be used as an antidiuretic agent.

As shown in Fig. 8 and Fig. 9, the peptides of the present invention strongly suppress the growth of renal epithelial cells via a calcitonin receptor. It has been reported that calcitonin strongly suppresses the growth of adenocarcinoma cells, etc. via a calcitonin receptor (*Cancer Res.* 1985, 45, 4890-4894 and others). Accordingly, it is expected that the peptides of the present invention will similarly suppress the growth of cancer cells via a calcitonin receptor in a stronger manner than calcitonin and they are able to be used as treating and preventive agents for cancer.

It was detected the large number of the peptides of the present invention in the central nervous system. With regard to calcitonin already known in the prior art as an agonist, its expression in the central nervous system is not detected while calcitonin receptor is expressed in the central nervous system and such a fact shows that the peptide of the present invention is a genuine endogenous ligand in the central nervous system. It has been reported that, when calcitonin is administered into a cerebral ventricle, suppression of appetite and analgesic action are resulted. Since calcitonin is not expressed in the brain, it is believed that the present peptides carry such an action in the brain.

Therefore, it is believed that the peptides of the present invention act on calcitonin receptor in the central nervous system and elicit the following pharmaceutical effects.

It has been reported that calcitonin acts for suppressing the appetite in the central nervous system (*Science* 1979, 206, 850-852; *The Bone* 1992, 6, 69-74; etc.). The peptides of the present invention, which can activate a calcitonin receptor more strongly than calcitonin, have a great possibility to elicit a strong appetite-suppressing action. The peptides of the present invention, by means of administration, can be used as a treating and preventive agent for obesity and diseases caused thereby (such as hypertension and hyperlipemia).

It has been further reported that calcitonin preparations show analgesic action to pain of bone due to metastasis of cancer, osteoporosis, etc., to pain by migraine and pancreatitis, etc. (*Am. J. Med. Sci.* 1997, 313, 13-16; etc.). There is a high possibility that the peptides of the present invention, which activate the calcitonin receptor morestrongly than calcitonin, have a strong analgesic action same as calcitonin and they are effective as analgesics to those pains.

The peptides of the present invention have characteristics of: (1) being expressed in central nervous systems, (2) strongly acting on calcitonin receptor, (3) promoting a cAMP productivity of cells, and relate to the peptides which have at least the following amino acid sequence Ser-Cys-Asn-Thr-Ala-Thr-Cys-Met-Thr-His-          10 Arg-Leu-Val-Gly-Leu-Leu-Ser-Arg-Ser-Gly-          20 Ser-Met-Val-Arg-Ser-Asn-Leu-Leu-Pro-Thr-          30 Lys-Met-Gly-Phe-Lys-Val-Phe-Gly 38 or an amino acid sequence where a part of the amino acids are deleted, substituted or added. The peptides of the present invention include amide derivatives, ester derivatives and derivatives of functional group of side chain of each amino acid such as amino group of lysine and hydroxyl group of serine provided that the above described amino acid sequence is comprised. More preferable peptides of the present invention further have the characteristics of: (4) incorporating sodium ion concentration-dependently, (5) suppressing the incorporation of calcium ion, (6) suppressing the cell proliferation.

The peptides of the present invention are not limited to the above described amino acid sequence so far as they have the above described characteristics (1) to (3) or, preferably, (1) to (6), and they may be peptides where a part of the amino acids in the amino acid sequence are deleted, substituted and/or added. With regard to the amino acid sequence as such, though not limited to, it preferably has 50% or more homology, more preferably may have 75% or more, 80% or more, still more preferably may as well have 85% or more homology. Furthermore, the preferable peptides of the present invention include, those which comprising an amino acid sequence having 50% or more, preferably 60% or more homology to the amino acid sequence of calcitonin or calcitonin gene-related peptides.

In the above example, CRSPs of the present invention derived from a pig have been described, however, the peptides of the present invention are not limited to those derived from pig but comprise the peptides derived from mammals such as human beings, rats, dogs and cows and fish such as salmon, etc.

The peptides of the present invention can be separated or purified from natural products by a manner such as extraction, or can be synthesized by way of common peptide synthesis methods. It is further possible to produce the peptide by means of a gene recombination technique where the gene of the present invention is incorporated into a suitable vector, and prokaryotic cells or eukaryotic cells are used as hosts.

The pharmaceutical composition of the present invention comprises the peptide of the present invention and a pharmaceutically acceptable carrier. The pharmaceutical composition of the present invention can be administered either orally or parenterally, and preferably it is administered parenterally by such as intravenous injection and intramuscular injection.

The pharmaceutical composition of the present invention can be made into preparations such as a preparation for injection, a freeze-dried preparation and a preparation for rectal administration. It also can be made into preparations for external application such as adhesive device, ointment and plaster. It can further be made into preparations such as sublingual tablets. Formulation can be carried out by means of general methods for peptide preparation.

The pharmaceutical composition of the present invention can be used in combination with other effective ingredients other than the peptide of the invention. With regard to the dose of the pharmaceutical composition of the present invention, it can be administered usually within a range from 1 µg/kg to 1,000 mg/kg of body weight, preferably from 0.1 mg/kg to 500 mg/kg of body weight per day in terms of the amount of the peptides of the present invention, however, may be varied depending on nature of the patient and condition of the disease. Since the peptide of the present invention acts more on calcitonin receptor than calcitonin does, it may be applied to the diseases in the same manner as in the case of the conventional calcitonin preparations.

Since the peptide of the invention further includes a peptide having identity with a calcitonin gene-related peptide (CGRP), it can be utilized not only for the above described object but a physiological activity thereof similar to CGRP can also be utilized.

The known physiological activity of CGRP include such as acting strongly on a CGRP receptor and promoting the cAMP production in cells. It also has been reported that CGRP induces relaxation of blood vessel (*Regul. Pept.* 1986, 15, 1-23; etc.), promotes diuresis (*Proc. Soc. Exp. Biol. Med.* 1998, 188, 316-322; etc.) and alters the proliferation potency of vascular endothelial cells and cells such as vascular smooth muscle and fibroblast (*Proc. Natl. Acad. Sci. USA* 1990, 87, 3299-3303; *Regul. Pept.* 2001, 101, 169-178; etc.).

Accordingly, with regard to some of the peptides of the present invention, particularly those having a high homology to CGRP, it is expected to have function of (1) being expressed in central nervous system, (2) strongly acting on a calcitonin receptor, (3) promoting a cAMP productivity of cells, (4) incorporating sodium ion concentration-dependently, (5) suppressing the incorporation of calcium ion, and (6) suppressing the cell proliferation. In addition to the above, it is also expected to have the similar functions to those of CGRP such as (7) strongly acting on calcitonin-related peptide receptor, (8) inducing relaxation of bloodvessel, (9) promoting diuresis, and (10) altering the proliferation potency of vascular endothelial cells, vascular smooth muscle and fibroblast, although all the peptides of the present invention do not always have those functions of (7) to (10).

According to the functions that CGRP have, the pharmaceutical composition containing the peptide of the present invention can be utilized as preparations in use for the following purposes in addition to the above described usages. Namely, it can be used for such as a hypotensive agent where the function of blood vessel relaxation is directly utilized, a hypotensive agent where the function of blood vessel relaxation via central and peripheral nervous systems is utilized, a diuretic, and preventives for restenosis after PTCA (percutaneous transluminal coronary angioplasty) where the function of suppressing the proliferation of smooth muscle of blood vessel is utilized.

Meanwhile, all the contents disclosed in the specification of Japanese Patent Application No. 2002/162, 797 will be incorporated herein.

### Examples

The present invention will now be more specifically illustrated by way of the following Examples although the present invention is never limited by those Examples.

### Example 1 (Extraction and isolation of CRSP peptide)

Pig brain (20 kg) was boiled for 10 minutes in water of a 4-fold amount, cooled and homogenized by addition of acetic acid as to make its final concentration 1M and the insoluble matters were removed by centrifugation to prepare a pig brain extract. This was desalted and concentrated by means of ultrafiltration (Pericon Cassette PLAC #000-05, manufactured by Millipore) and acetone was gradually added to the extract after desalting under cooling with ice so as to make the final concentration 66%. The precipitate was removed by centrifugal separation, acetone was removed from the supernatant liquid using an evaporator and the residue was adsorbed with a reversed phase column (LC-SORB SPW-C-ODS; Chemco; 1.5 l). After the column was washed with a 3-fold amount of 0.5 M acetic acid, the peptide fraction was eluted with a 3-fold amount of eluting buffer (water : acetonitrile : 10% trifluoroacetic acid = 40:60:1). Acetonitrile was removed from the eluate using an evaporator followed by subjecting to a freeze-drying.

The freeze-dried specimen was weighed, dissolved in 1 M acetic acid, adsorbed with an open column filled with a cation-exchange resin (SP-Sephadex, Pharmacia, 3 × 28 cm) and fractionated into a fraction (SP-I) which just passed therethrough (SP-I), a fraction (SP-II) eluted with 2 M pyridine (pH8.0) and a fraction (SP-III) eluted with 2 M pyridine-acetic acid (pH 5.0) to prepare an SP-III strongly ionic fraction. The fraction was freeze-dried, dissolved in 1 M acetic acid and fractionated into fractions corresponding to molecular weights by means of gel filtration (Sephadex G-50, Pharmacia, 7.5 × 145 cm, 1 M acetic acid, flow rate: 100 ml/hour) whereupon the part corresponding to molecular weights of from 1 kDa to 5 kDa was collected. It was again subjected to gel filtration (Sephadex G-25, Pharmacia, 7.5 × 145 cm, 1 M acetic acid, flow rate: 100 ml/hour) to separate into fractions corresponding to molecular weights and the part corresponding to molecular weights of from 2 kDa to 4 kDa was collected and freeze-dried.

The freeze-dried specimen was subjected to a concentration-gradient elution by ammonium formate of from 10 mM to 0.5 M using ion-exchange chromatography (CM52, Whatman, 2.4 × 45 cm; solution A: 10 mM ammonium formate (pH 6.5) : acetonitrile = 9:1; solution B: 1 M ammonium formate (pH 6.5) : acetonitrile = 9:1; flow rate: 35 ml/hour).

Then a 1/1,000 amount of each fraction was freeze-dried, LLC-PK₁ (epithelial cells derived from kidney of pig) was stimulated by a medium where the freeze-dried specimen was dissolved in a Dulbecco's modified Eagle medium (DMEM, containing 0.05% of bovine serum albumin (BSA)), incubation was conducted at 37°C for 1 hour and amount of cAMP secreted to the medium was quantified by radioimmunoassay.

The method therefor is as follows. Thus, an equivalent amount of a mixed solution of dioxane-triethylamine (dioxane : triethylamine = 4:1) dissolved in succinic acid anhydride so as to make 4% was added to 100 µl of a medium containing an unknown amount of cAMP and a medium containing a known amount of cAMP, the mixture was allowed to stand at room temperature for 30 minutes so that cAMP was succinylated and evaporated to dryness using a centrifugal evaporator, the residue was dissolved in 1 ml of buffer (50 mM sodium acetate (pH 6.2), 1 mM EDTA, 0.025% of sodium azide, 0.5% serum albumin and 0.01% Triton X-100), each 100 µl thereof was taken out to a test tube and a radio-labeled succinylated cAMP and antibody were added to each 50 µl followed by being allowed to stand at 4°C for 48 hours. After that, 100 µl of 1% γ-globulin and 500 µl of 25% polyethylene glycol were added thereto, the mixture was well mixed and centrifuged and radioactivity of the precipitate was measured by a gamma-counter and compared with radioactivity of the standard curve prepared by cAMP of the known concentration whereupon the amount of cAMP was quantified.

The fraction where a rise of cAMP amount was observed (about 0.3 M of ammonium formate (pH 6.5)) was further subjected to a concentration-gradient elution with ammonium formate using a cation-exchange HPLC (TSK-gel CM-2SW, Tosoh, 7.8 × 300 mm; solution A: 10 mM ammonium formate (pH 3.8) : acetonitrile = 9:1; solution B: 1 M ammonium formate (pH 3.8) : acetonitrile = 9:1; flow rate: 2 ml/min). Again, a 1/1,000 amount of each fraction was taken out, its activity was measured, the fraction where activity was observed (about 0.36 M of ammonium formate (pH 3.8)) was further subjected to a concentration-gradient elution by acetonitrile using a reversed phase HPLC (C₁₈ 218TP54, Vydac, 4.6 × 250 mm; solution A: water : acetonitrile : 10% trifluoroacetic acid = 90:10:1; solution B: water : acetonitrile : 10% trifluoroacetic acid = 40:60:1); flow rate: 1 ml/min) and a fraction having activity (about 32% of acetonitrile) was collected. This was again separated by a reversed phase HPLC (diphenyl 219TP5215, Vydac, 2.1 × 150 mm; solution A: water : acetonitrile : 10% trifluoroacetic acid = 90:10:1; solution B: water : acetonitrile : 10% trifluoroacetic acid = 40:60:1; flow rate: 0.2 ml/min) whereupon a final pure specimen (about 29% of acetonitrile) was prepared.

A peptide (about 50 pmol) was prepared. Molecular weight of this peptide was 4,099 and a theoretical isoelectric point was calculated to be 12.81. In addition, it had such a hydrophobic property that was able to be eluted with acetonitrile of about 32% concentration in the presence of 0.1% of trifluoroacetic acid in a C₁₈ column (4.6 × 250 mm, 218TP54) of Vydac as shown above.

### Example 2 (Determination of amino acid sequence of CRSP)

An amino acid sequence was determined by an Edman method using an automatic amino acid sequencer. Firstly, 5 pmol of the pure specimen was analyzed by the amino acid sequencer from its N-terminal and the following was determined.

Ser-Xaa-Asn-Thr-Ala-Thr-Xaa-Met-Thr-His-Arg-Leu-Val-Gly-Leu-Leu-Ser-Arg-Ser-Gly-Ser-Met-Val

After that, 10 pmol of the pure specimen was subjected to a trypsin degradation and subjected to a concentration-gradient elution by acetonitrile using a reversed phase HPLC (C₁₈ 218TP5215, Vydac, 2.1 × 150 mm; solution A: water : acetonitrile : 10% trifluoroacetic acid = 90:10:1; solution B: water : acetonitrile : 10% trifluoroacetic acid = 40:60:1; flow rate: 0.2 ml/min). The resulting peaks were analyzed by the amino acid sequencer whereupon the following six sequences were able to be obtained.

Ser-Xaa-Asn-Thr-Ala-Thr-Xaa-Met-Thr-His-Arg,

Leu-Val-Gly-Leu-Leu-Ser-Arg,

Ser-Gly-Ser-Met-Val-Arg,

Ser-Asn-Leu-Leu-Pro-Thr-Lys,

Met-Gly-Phe-Lys and

Val-Phe

As a result of comparison of those sequences with the databases, it was concluded that the present peptide has a homology to a calcitonin gene-related peptide and has the following sequence.

Ser-Cys-Asn-Thr-Ala-Thr-Cys-Met-Thr-His-Arg-Leu-Val-
Gly-Leu-Leu-Ser-Arg-Ser-Gly-Ser-Met-Val-Arg-Ser-Asn-
Leu-Leu-Pro-Thr-Lys-Met-Gly-Phe-Lys-Val-Phe-NH2

In the meanwhile, its molecular weight was measured using a mass spectrometer and the result was 4,130.6 ± 0.7 Da. This was different from 4,042 Da presumed from the amino acid sequence to an extent of about 89 Da but the difference is presumed to be due to oxidation of two methionines (2 × 16 Da) and the presence of glycine (57 Da) at C-terminal which was not able to be measured by the amino acid sequencer. Finally, from the next Example 3, it was found to be as follows which was identical with those presumptions. Ser-Cys-Asn-Thr-Ala-Thr-Cys-Met-Thr-His-Arg-Leu-Val- Gly-Leu-Leu-Ser-Arg-Ser-Gly-Ser-Met-Val-Arg-Ser-Asn- Leu-Leu-Pro-Thr-Lys-Met-Gly-Phe-Lys-Val-Phe-Gly-NH2 (A disulfide bond is formed between the second Cys and the seventh Cys.)

An amino acid sequence of the resulting CRSP is shown in SEQ ID NO: 1 of Sequence Listing while an amino acid sequence of CRSP-Gly is shown in SEQ ID NO: 2.

### Example 3 (Complementary DNA base sequence and precursor amino acid sequence)

A probe was prepared by formation of a synthetic primer using 
(TG(C/T)AA(C/T)AC(A/C/G/T)GC(A/C/G/T)AC(A/C/G/T)TG(C
/T)ATGAC)
 of the N-terminal side and (CC(A/G)AA(A/C/G/T)AC(C/T)TT(A/G)AA(A/C/G/T)CCCATA) of the C-terminal side on the basis of the amino acid sequence of Example 2 and by amplification using a PCR where pig gene was used as a template.

A complementary DNA λ phage library was prepared from pig hypothalamus mRNA (3 µg) using a Time Saver cDNA preparing kit of Pharmacia and λ ZAPII of Stratagene. A method for the screening was as follows. Thus, *Escherichia coli* was infected to λ phage (having about 100,000 independent clones), mixed with 0.7% agarose containing an LB medium, sown on an incubation dish laid with a 1.5% agar medium containing an LB medium and incubated for about 8 hours at 37°C. After it was cooled in a refrigerator for about 2 hours, the plaque formed thereby was transcribed to a Nylon filter, made into alkaline (by treating for 2 minutes with a 0.5 M sodium hydroxide and 1.5 M sodium chloride solution; for 2 minutes with a 0.5 M Tris-hydrochloride (pH 7.5) and 1.5 M sodium chloride solution; and for 5 minutes with a 45 mM sodium citrate (pH 7.0) and 450 mM sodium chloride solution) and then the filter was dried at 80°C for 2 hours. After that, the dried filter was dipped for at 37°C 2 hours in a pre-hybridization solution (50% of formamide, 0.09 M of sodium citrate (pH 7.0), 0.9 M of sodium chloride, 0.5% of bovine serum albumin, 0.5% of Ficoll, 0.5% of polyvinylpyrrolidone and 0.5% of sodium laurylsulfate), a probe labeled with ³²P was added thereto and the mixture was subj ected to hybridization at 42°c for 16 hours. After the filter was washed with washing solutions (with a solution of 30 mM of sodium citrate (pH 7.0), 300 mM of sodium chloride and 0.1% of sodium laurylsulfate at room temperature for 5 minutes for two times and with a solution of 3 mM of sodium citrate (pH 7.0), 30 mM of sodium chloride and 0.1% of sodium laurylsulfate at 65°C for 1 hour for two times), it was exposed to X-ray film, the area sensitized with radioactive ray was compared with the plaque on the culture dish and positive clone was isolated from the plaque.

The isolated positive λ phage clone was converted to a plasmid pBluescript which is a vector suitable for a DNA sequencing using Helper Phage R408 of Stratagene and a nucleotide sequence was determined by a Sanger method.

The determined base sequence is shown in SEQ ID NO: 3 of the Sequence Listing while its amino acid sequence is shown in SEQ ID NO: 4.

The resulting CRSP has a homology in terms of the amino acid sequence topig calcitonin (CT) gene-related peptide (CGRP), human CGRP-I and human CGRP-II to an extent of 71.1%, 63.2% and 71.1%, respectively (refer to Fig. 2).

### Example 4 (Expression site)

Preparation of RNA for expressed amount of gene was carried out by means of extracting with acidic guanidine, phenol and chloroform. About 1 g of pig tissue was homogenized with 5 ml of a denaturing solution (4 M of guanidine thiocyanate, 25 mM of sodium citrate (pH 7.0), 0.1 M of 2-mercaptoethanol and 0.5% of sodium sarcosinate), well stirred with a 1/10 amount of 2 M sodium acetate, an equivalent amount of water-saturated phenol and a 1/5 amount of chloroform and the mixture was subjected to a centrifugal separation. After that, an aqueous layer was separated, the same amount of 2-propanol was added thereto and the mixture was well stirred, allowed to stand at -20°C for 1 hour and centrifuged to recover the precipitate (RNA). 30 µg of this RNA were subjected to an electrophoresis using a formaldehyde-agarose modified gel (1% of agarose, 2.2 M of formaldehyde, 20 mM of Mops (pH 7), 8 mM of sodium acetate and 1 mM of EDTA), the gel after the electrophoresis was well washed with water to remove formaldehyde and then treated with 0.05 M of sodium hydroxide for 15 minutes and a solution of 0.3 M of sodium citrate (pH 7) and 3 M of sodium chloride for 45minutes. Afterthat, RNA contained in the gel was transferred to a Nylon filter by a capillary blot method and the filter to which RNA was transcribed was dried at 80°C. After that, the filter was dipped in a ULTRAhyb hybridization solution of Ambion at 37°C for 2 hours, a probe labeled with ³²P was added where CRSP was encoded (a base pair between 346th to 488th ones on cDNA) and hybridization was conducted at 42°C for 16 hours. After the hybridization, the filter was washed with a washing solution (with a solution of 30 mM of sodium citrate (pH 7.0), 300 mM of sodium chloride and 0.1% of sodium laurylsulfate at room temperature for 5 minutes for two times and with a solution of 1.5 mM of sodium citrate (pH 7.0), 15 mM of sodium chloride and 0.1% of sodium laurylsulfate at 65°C for 1 hour for two times) and then exposed to an X-ray film whereupon RNA was quantified.

The result is shown in Fig. 3.

### Example 5 (Measurement of expressed amount)

Amount of peptide in each tissue was measured by radioimmunoassay utilizing an antibody prepared to the synthetic peptide. The tissue (about 1 g) was boiled with water in a 10-fold amount and cooled with ice, acetic acid was added so as to make it 1 M, homogenization was conducted and insoluble matter were removed by centrifugal separation to prepare an extract. The extract was freeze-dried and dissolved in a buffer (50 mM of sodium phosphate (pH 7.4), 80 mM of sodium chloride, 25 mM of EDTA, 0.5% of Triton X-100, 0.5% of bovine serum albumin and 0.05% of sodium azide) and each 100 µl thereof were taken out to a test tube. To a buffer containing CRSP as such and to a buffer in which a known concentration of CRSP were added the same amount of radiolabeled CRSP and antibody, the mixture was stirred and allowed to stand at 4°C for 48 hours, 100 µl of γ-globulin and 500 µl of 23% polyethylene glycol were added thereto, the mixture was well mixed and centrifuged and radioactivity of the precipitate was compared with radioactivity of a standard solution prepared from CRSP of known concentration whereupon the amount of CRSP was quantified.

The result is shown in the above Table 1.

### Example 6 (Promoting action of CRSP for cAMP production in LLC-PK₁ cells)

LLC-PK₁ cells were incubated in a culture medium (a Dulbecco-modified Eagle medium (DMEM) containing 10% of fetal calf serum (FCS)), CRSP dissolved in DMEM containing 0.05% of bovine serum albumin was substituted for a culture medium for the cells after two days, incubation was conducted for 30 minutes, the medium was recovered and amount of the secreted cAMP was quantified by radioimmunoassay using a cAMP-specific antibody.

The result is shown in Fig. 4.

### Example 7 (Action for incorporation of sodium ion and calcium ion in the presence of CRSP in LLC-PK₁ cells)

LLC-PK₁ cells were incubated for two days in a DMEM (containing 10% of FCS) on a six-well incubation dish. Measurement of incorporation of sodium ion was conducted as follows.

An incubation solution for LLC-PK₁ cells was substituted with a Hanks' choline chloride solution, sodium chloride (²²Na) having a predetermined amount of radioactivity and CRSP were added thereto so as to make the final concentration 0 M, 10⁻⁸ M, 10⁻⁷ M and 10⁻⁶ M, respectively, the cells were washed after 10 minutes to completely remove ²²Na contained in the solution outside the cells and radioactivity incorporated into the cells was measured by a gamma-counter. Similarly, an incubated solution for LLC-PK₁ cells was substituted with a Hanks' choline chloride solution, sodium chloride (²²Na) havingapredetermined radioactivity and each of CRSP and an amiloride derivative (5-(N-ethyl-N-isopropyl)-amiloride) (EIPA) which is an Na/H co-transporter inhibitor were added so as to make the final concentration 0 M of CRSP + 0 M of EIPA, 10⁻⁶ M of CRSP + 0 M of EIPA, 10⁻⁶ M of CRSP + 10⁻⁸ M of EIPA, 10⁻⁶ M of CRSP + 10⁻⁷ M of EIPA, 10⁻⁶ M of CRSP + 10⁻⁶ M of EIPA and 0 M of CRSP + 10⁻⁶ M of EIPA, the cells were washed after 10 minutes to completely remove ²²Na contained in the solution outside the cells and radioactivity incorporated into the cells was measured by a gamma-counter.

The result in the absence of EIPA is shown in Fig. 5 while the result in the presence of EIPA is shown in Fig. 6.

Incorporation of calcium ion was conducted as follows. Thus, LLC-PK₁ cells were washed with a Hanks' solution (calcium-free Hanks' solution) where concentration of calcium chloride was lowered to 0 mM. Calcium chloride (⁴⁵Ca) was added to a calcium-free Hanks' solution on the cells together with CRSP, the cells were washed after 10 minutes to completely wash out ⁴⁵Ca contained in the solution outside the cells and radioactivity incorporated into the cells was measured by a liquid scintillation counter.

The result is shown in Fig. 7.

### Example 8 (Suppression of cell proliferation by CRSP)

LLC-PK₁ cells were incubated for two days in a DMEM (containing 10% of FCS) on a 24-well collagen plate, once washed with a DMEM and substituted with a DMEM (containing 10% of FCS) containing 0 and 10⁻¹² to 10⁻⁶ M of CRSP followed by incubating. After 2 hours, a DMEM (containing 0.1% of BSA) containing ¹²⁵I-labeled bromodeoxyuridine was added to each well and, after 5 hours, radioactivity incorporated into the nuclei was measured by a gamma-counter whereupon the amount of DNA synthesized as a result of cell proliferation was measured.

The result is shown in Fig. 8.

Similarly, LLC-PK₁ cells of 10,000 cells/well were sown on a 24-well collagen plate and incubated with a DMEM (containing 10% of FCS) for 24 hours. Each well was once washed with a DMEM, the medium was substituted with a DMEM (containing 10% of FCS) containing CRSP of concentrations of 0 M, 10⁻⁸ M and 10⁻⁶ M and incubation was conducted. After 24 hours, the cells on the incubation dish were peeled off with a trypsin-EDTA solution and cell numbers were counted by a counting plate.

The result is shown in Fig. 9.

### Example 9 (Action of CRSP on calcitonin receptor)

Pig calcitonin receptor (CTR) was introduced into a mammalian expression vector pcDNA 3.1 and expressed in rhesus monkey renal cells (COS-7) and production amount of cAMP by CRSP stimulation was measured by radioimmunoassay.

The result is shown in Fig. 10.

Similarly, CTR was expressed in opossum renal cells (OK cells) and production amount of cAMP by CRSP stimulation was measured by radioimmunoassay while incorporation of sodium ion was measured by incorporation of radioactive sodium chloride (²²Na) using a gamma-counter.

The results are shown in Fig. 11 and Fig. 12, respectively.

### Example 10 (Changes in blood pressure and ion concentration in plasma of rats by CRSP)

CRSP corresponding to the amount of 16 nmol/kg dissolved in a physiological saline solution was administered at a time within short period from a carotid artery of rat under an anesthetized condition.

The results are shown in Fig. 13 and Fig. 14.

### Example 11 (Extraction and isolation of CRSP-Gly peptide)

Pig brain (20 kg) was boiled for 10 minutes in water of a 4-fold amount, cooled and homogenized by addition of acetic acid to as to make its final concentration 1M and the insoluble matters were removed by centrifugation to prepare a pig brain extract. This was desalted and concentrated by means of ultrafiltration (Pericon Cassette PLAC #000-05, manufactured by Millipore) and acetone was gradually added to the extract after desalting under cooling with ice so as to make the final concentration 66%. The precipitate was removed by centrifugal separation, acetone was removed from the supernatant liquid using an evaporator and the residue was adsorbed with a reversed phase column (LC-SORB SPW-C-ODS; Chemco; 1.5 l). After the column was washed with a 3-fold amount of 0.5 M acetic acid, the peptide fraction was eluted with a 3-fold amount of eluting buffer (water : acetonitrile : 10% trifluoroacetic acid = 40:60:1). Acetonitrile was removed from the eluate using an evaporator followed by subjecting to a freeze-drying. The freeze-dried specimen was weighed, dissolved in 1 M acetic acid, adsorbed with an open column filled with a cation-exchange resin (SP-Sephadex, Pharmacia, 3 × 28 cm) and fractionated into a fraction (SP-I) which just passed therethrough (SP-I), a fraction (SP-II) eluted with 2 M pyridine (pH 8.0) and a fraction (SP-III) eluted with 2M pyridine-acetic acid (pH5.0) to prepare an SP-III strongly ionic fraction. The fraction was freeze-dried, dissolved in 1 M acetic acid and fractionated into fractions corresponding to molecular weights by means of gel filtration (Sephadex G-50, Pharmacia, 7.5 × 145 cm, 1 M acetic acid, flow rate: 100 ml/hour) whereupon the part corresponding to molecular weights of from 1 kDa to 5 kDa was collected. It was again subjected to gel filtration (Sephadex G-25, Pharmacia, 7.5 × 145 cm, 1 M acetic acid, flow rate: 100 ml/hour) to separate into fractions corresponding to molecular weights and the part corresponding to molecular weights of from 2 kDa to 4 kDa was collected and freeze-dried.

The freeze-dried specimen was subjected to a concentration-gradient elution by ammonium formate of from 10 mM to 0.5 M using ion-exchange chromatography (CM52, Whatman, 2.4 × 45 cm; solution A: 10 mM ammonium formate (pH 6.5) : acetonitrile = 9:1; solution B: 1 M ammonium formate (pH 6.5) : acetonitrile = 9:1; flow rate: 35 ml/hour).

Then a 1/1,000 amount of each fraction was freeze-dried, LLC-PK₁ (epithelial cells derived from kidney of pig) was stimulated by a medium where the freeze-dried specimen was dissolved in a Dulbecco's modified Eagle medium (DMEM, containing 0.05% of bovine serum albumin), incubation was conducted at 37°C for 1 hour and amount of cAMP secreted to the medium was quantified by radioimmunoassay.

The method therefor is as follows. Thus, an equivalent amount of a mixed solution of dioxane-triethylamine (dioxane : triethylamine = 4:1) dissolved in succinic acid anhydride so as to make 4% was added to 100 µl of a medium containing an unknown amount of cAMP and a medium containing a known amount of cAMP, the mixture was allowed to stand at room temperature for 30 minutes so that cAMP was succinylated and evaporated to dryness using a centrifugal evaporator, the residue was dissolved in 1 ml of buffer (50 mM of sodium acetate (pH 6.2), 1 mM of EDTA, 0.025% of sodium azide, 0.5% of serum albumin and 0.01% of Triton X-100), each 100 µl thereof was taken out to a test tube and a radiolabeled succinylated cAMP and antibody were added to each 50 µl followed by being allowed to stand at 4°C for 48 hours. After that, 100 µl of 1% γ-globulin and 500 µl of 25% polyethylene glycol were added thereto, the mixture was well mixed and centrifuged and radioactivity of the precipitate was measured by a gamma-counter and compared with radioactivity of the standard curve prepared by cAMP of the known concentration whereupon the amount of cAMP was quantified.

A rise in the cAMP production caused by CRSP-Gly was observed in a fraction eluted by about 0. 27 M of ammonium formate (pH 6.5). A concentration-gradient elution with ammonium formate was further conducted using a cation-exchange HPLC (TSK-gel CM-2SW, Tosoh, 7.8 × 300 mm; solution A: 10 mM ammonium formate (pH 3.8) : acetonitrile = 9:1; solution B: 1 M ammonium formate (pH 3.8) : acetonitrile = 9:1; flow rate: 2 ml/min). Again, a 1/1,000 amount of each fraction was taken out, its activity was measured, the fraction where activity was observed (being eluted with about 0.36 M of ammonium formate (pH 3.8)) was further subjected to a concentration-gradient elution by acetonitrile using a reversed phase HPLC (C₁₈ 218TP54, Vydac, 4.6 × 250 mm; A solution: water : acetonitrile : 10% trifluoroacetic acid = 90:10:1; B solution: water : acetonitrile : 10% trifluoroacetic acid = 40:60:1; flow rate: 1 ml/min) and a fraction having activity (about 32% of acetonitrile) was collected. This was again separated by a reversed phase HPLC (diphenyl 219TP5215, Vydac, 2. 1 × 150 mm; solution A: water : acetonitrile : 10% trifluoroacetic acid = 90:10:1; solution B: water : acetonitrile : 10% trifluoroacetic acid = 40:60:1; flow rate: 0.2 ml/min) whereupon a final pure specimen (being eluted with about 29% of acetonitrile) was prepared.

In the actual purifying step, about 50 pmol of peptide were prepared. Molecular weight of this peptide was 4, 157 Da and a theoretical isoelectric point was calculated to be 11.41. In addition, it had such a hydrophobic property that it was able to be eluted with acetonitrile of about 32% concentration in the presence of 0.1% of trifluoroacetic acid in a C18 column (4.6 × 250 mm, 218TP54) of Vydac as shown above.

### Example 12 (Action for promotion of cAMP production by CRSP-Gly)

Pig calcitonin receptor (CTR) was introduced into a mammalian expression vector pcDNA 3.1, then expressed in rhesus monkey renal cells (COS-7) and production amount of cAMP by CRSP or CRSP-Gly stimulation was measured by radioimmunoassay.

The result is shown in Fig. 15.

### Example 13 (cDNA cloning of canine and bovine CRSP)

RNA was extracted from canine and bovine thyroid gland by a method using acidic guanidine, phenol and chloroform. Purification of mRNA was conducted using an Oligotex-dT30 mRNA purifying kit of Takara Shuzo. Complementary DNA λ phage library was prepared from canine and bovine thyroid gland mRNA (3 µg) using a Time Saver cDNA preparation kit of Pharmacia and a λ ZAPII of Stratagene. With regard to a probe, a pig calcitonin receptor-stimulating peptide cDNA having a full length of 700 bp was used. A method for the screening was as follows. Thus, *Escherichia coli* was infected to λ phage (having about 300,000 independent clones), mixed with 0.7% agarose containing an LB medium, sown on an incubation dish laid with a 1.5% agar medium containing an LB medium and incubated for about 8 hours at 37°C. After it was cooled in a refrigerator for about 2 hours, the plaque formed thereby was transferred to a Nylon filter, made into alkaline (by treating for 2 minutes with a 0. 5 M sodium hydroxide and 1.5 M sodium chloride solution; for 2 minutes with a 0.5 M Tris-hydrochloride (pH 7.5) and 1.5 M sodium chloride solution; and for 5 minutes with a 45 mM sodium citrate (pH 7.0) and 450 mM sodium chloride solution) and then the filter was treated at 80°C for 2 hours. After that, the filter was dipped at 37°C for 2 hours in a pre-hybridization solution (20% of formamide, 0.09 M of sodium citrate (pH 7.0), 0.9 M of sodium chloride, 0.5% of bovine serum albumin, 0.5% of Ficoll, 0.5% of polyvinylpyrrolidone and 0.5% of sodium laurylsulfate), a probe labeled with ³²P was added thereto and the mixture was subjected to hybridization at 42°c for 16 hours. After the filter was washed with washing solutions (with a solution of 30 mM of sodium citrate (pH 7.0), 300 mM of sodium chloride and 0.1% of sodium laurylsulfate at room temperature for 5 minutes for two times and with a solution of 15 mM of sodium citrate (pH 7.0), 150 mM of sodium chloride and 0.1% of sodium laurylsulfate at 60°C for 1 hour for two times), it was exposed to X-ray film, the area sensitized with radioactive ray was compared with the plaque on the culture dish and positive clone was isolated from the plaque. The isolated positive λ phage clone was converted to a plasmid pBluescript which is a vector suitable for a DNA sequencing using Helper Phage R408 of Stratagene and a nucleotide sequence was determined by a Sanger method.

A nucleotide sequence of cDNA coding for the determined bovine CRSP is shown in SEQ ID NO: 7 of the Sequence Listing which will be given later and an amino acid sequence of a precursor peptide of the coded bovine CRSP is shown in SEQ ID NO: 8. An amino acid sequence of bovine CRSP is shown in SEQ ID NO: 6.

A nucleotide sequence of cDNA coding for the determined canine CRSP is shown in SEQ ID NO: 10 of the Sequence Listing which will be given later and an amino acid sequence of a precursor peptide of the coded canine CRSP is shown in SEQ ID NO: 11. An amino acid sequence of canine CRSP is shown in SEQ ID NO: 9.

### Example 14 (Quantification of production amount of cAMP by stimulation with bovine CRSP or canine CRSP)

LLC-PK₁ cells were incubated in a culture medium (a Dulbecco-modified Eagle medium (DMEM) containing 10% of fetal bovine serum). After two days, the cells were washed twice with a DMEM containing 0.05% of bovine serum albumin, the medium in the incubation dish was substituted with a DMEM containing 0. 5 mM of 3-isobutyl-1-methylxanthine and 0.05% of bovine serum albumin (DMEM/BSA/IBMX) and incubation was conducted at 37°C for 30 minutes. After the incubation, the medium was substituted with a DMEM/BSA/IBMX in which CRSP was dissolved so as to make it 0 M and 10⁻¹² to 10⁻⁶ M and incubation was conducted at 37°C for 10 minutes. After the incubation, the medium in the incubating dish was removed, ethanol was added thereto and the incubating dish was once frozen in a freezer for the cell lysis. Then ethanol was transferred to a test tube and the specimen was evaporated to dryness using a centrifugal evaporator. The dried specimen was dissolved in a DMEM, each 100 µl thereof was taken out, the same amount of dioxane-triethylamine mixture (dioxane : triethylamine = 4:1) in which succinic acid anhydride was dissolved so as to make it 4% was added thereto and the mixture was allowed to stand at room temperature for 30 minutes so that cAMP was succinylated. It was again evaporated to dryness using a centrifugal evaporator, dissolved in 1 ml of buffer (50 mM of sodium acetate (pH 6.2), 1 mM of EDTA, 0.025% of sodium azide, 0.5% of serum albumin and 0.01% of Triton X-100), each 100 µl thereof was taken out into a test tube, 50 µl of a radiolabeled succinylated cAMP and 50 µl of antibody were added and the mixture was allowed to stand at 4°C for 48 hours. After that, 100 µl of 1% γ-globulin and 500 µl of 25% polyethylene glycol were added, the mixture was well mixed and centrifuged and radioactivity of the precipitate was measured using a gamma-counter and compared with radioactivity of the standard curve prepared by cAMP of known concentrations whereupon the amount of cAMP was quantified.

The result is shown in Fig. 16.

### Example 15 (Cloning of CRSP-2 and CRSP-3/CT-2 precursor genes)

Pig genomic library was purchased from Clontech. With regard to a probe, a precursor cDNA having a full length of 700 bp of a pig calcitonin receptor-stimulating peptide was used. Method for screening was that *Escherichia coli* was infected to λ phage (it has about 1,000,000 independent clones and about 300,000 thereof were used) into which pig genome was inserted, mixed with 0.7% agarose containing an LB medium, sown on an incubation dish where 1.5 % agar medium containing an LB medium was spread and incubated at 37°C for about 8 hours. After cooling in a refrigerator for about 2 hours, the resulting plaque was transferred to a Nylon filter and subjected to an alkaline modification (being treated with a 0.5 M of sodium hydroxide and 1.5 M of sodium chloride solution for 2 minutes, with a 0.5 M Tris-hydrochloride (pH 7.5) and 1. 5 M sodium chloride solution for 2 minutes and with a 45 mM of sodium citrate (pH 7.0) and 450 mM of sodium chloride solution for 5 minutes) and the filter was treated at 80°C for 2 hours. Then, the filter was dipped in a pre-hybridization solution (20% of formamide, 0.09 M of sodium citrate (pH 7.0), 0.9 M of sodium chloride, 0.5% of bovine fetal albumin, 0.5% of Ficoll, 0.5% of polyvinylpyrrolidone and 0.5% of sodium laurylsulfate) at 37°C for 2 hour, a probe labeled with ³²P was added thereto and hybridization was conducted at 42°C for 16 hours. After the filter was washed with washing solutions (with a 30 mM of sodium citrate (pH 7.0), 300 mM of sodium chloride and 0.1% of sodium laurylsulfate solution at room temperature for 5 minutes for two times and with a 7.5 mM of sodium citrate (pH 7.0), 75 mM of sodium chloride and 0.1% of sodium laurylsulfate solution at 55°C for 1 hour for two times), it was exposed to an X-ray film, the area which was sensitized with radioactive ray was compared with the plaque on the incubation dish and a positive clone was isolated from the plaque. The isolated positive λ phage clone was cleaved using various restriction enzymes, analysis was conducted by Southern blotting method to prepare a restriction enzyme map and DNA containing the positive clone was sub-cloned to pBluescript using an appropriate restriction enzyme. A nucleotide sequence was determined by a Sanger method. As a result of a screening, 25 positive clones were able to be prepared. As a result of analysis using restriction enzyme and determination of a nucleotide sequence, it was found that ten clones coded for gene of CRSP (Fig. 17). It was also found that 9 of the remaining 15 clones coded for other gene (Fig. 18 and Fig. 19) and six thereof coded for still other gene. The nine clones had sequences having a homology to CRSP. The genes were named CRSP-2. Other six clones had sequences having a homology to CRSP and CT. Those genes were named CRSP-3 and CT-2. In Fig. 17 to Fig. 19, the underlined parts show exons.

A nucleotide sequence of CRSP gene is shown in SEQ ID NO: 5 of the Sequence Listing which will be shown later. A CRSP-2 gene is shown in SEQ ID NO: 15 of the Sequence Listing. A nucleotide sequence of DNA fragment containing CRSP-3 gene and CT-2 gene is shown in SEQ ID NO: 23 of the Sequence Listing.

cDNA sequences which were presumed using the gene sequences of CRSP and CT/CGRP as references are shown in Fig. 20 to Fig. 22 (Fig. 20: CRSP-2; Fig. 21: CRSP-3; and Fig. 22: CT-2). In Fig. 20 to Fig. 22, the parts enclosed with solid lines are mature peptides, the gray parts are glycine which is presumed to be converted to amide and the *oblique parts* are signal peptides and, in Fig. 22, glutamine at the N-terminal side of the mature substance is presumed to be converted to pyroglutamic acid. Comparison of each CRSP with CGRP, AM, CT-2 and CT amino acids is Fig. 23.

A nucleotide sequence of cDNA of CRSP-2 is shown in SEQ ID NO: 13 of the Sequence Listing which will be given later, that of cDNA of CRSP-3 is shown in SEQ ID NO: 17 and that of cDNA of CT-2 is shown in SEQ ID NO: 21.

Mature amino acid sequences of CRSP-2, CRSP-3 and CT-2 are presumed to be as follows.

CRSP-2: Ser-Cys-Asn-Thr-Ala-Ser-Cys-Val-Thr-His 10
Lys-Met-Thr-Gly-Trp-Leu-Ser-Arg-Ser-Gly 20
Ser-Val-Ala-Lys-Asn-Asn-Phe-Met-Pro-Thr 30
Asn-Val-Asp-Ser-Lys-Ile-Leu-NH2 37

CRSP-3:    Ser-Cys-Asn-Thr-Ala-Ile-Cys-Val-Thr-His  10
           Lys-Met-Ala-Gly-Tro-Leu-Ser-Arg-Ser-Gly  20
           Ser-Val-Val-Lys-Asn-Asn-Phe-Met-Pro-Ile  30
           Asn-Met-Gly-Ser-Lys-Val-Leu-NH2          37

CT-2:    pGlu-Cys-Asn-Asn-Leu-Ser-Thr-Cys-Val-Leu 10
         Gly-Thr-Tyr-Thr-Trp-Asp-Val-Asn-Lys-Phe 20
         Tyr-Ala-Phe-Pro-Leu-Thr-Thr-Thr-Gly-Ile 30

Arg-Val-Ser-NH2 33

An amino acid sequence of CRSP-2 set forth in SEQ ID NO: 12 of the Sequence Listing, that of CRSP-3 is shown in SEQ ID NO: 16 and that of CT-2 is shown in SEQ ID NO: 19.

### Example 16 (cDNA cloning of a precursor of CRSP-2)

With regard to a cDNA library, there was used a λ phage library into which pig hypothalamus DNA was inserted being prepared in conducting a cDNA cloning of CRSP. With regard to a probe, a pig calcitonin receptor-stimulatingpeptide having a full length of 700 bp was used. Method for screening was that *Escherichia coli* was infected to λ phage into which pig hypothalamus DNA was inserted, mixed with 0.7% agarose containing an LB medium, sown on an incubation dish where 1.5 % agar medium containing an LB medium was spread and incubated at 37°C for about 8 hours. After cooling in a refrigerator for about 2 hours, the resulting plaque was transferred to a Nylon filter and subjected to an alkaline denaturation (being treated with a 0.5 M of sodium hydroxide and 1.5 M of sodium chloride solution for 2 minutes, with a 0.5 M Tris-hydrochloride (pH 7.5) and 1.5 M sodium chloride solution for 2 minutes and with a 45 mM of sodium citrate (pH 7.0) and 450 mM of sodium chloride solution for 5 minutes) and the filter was treated at 80°C for 2 hours. Then, the filter was dipped in a pre-hybridization solution (20% of formamide, 0.09 M of sodium citrate (pH 7.0), 0.9 M of sodium chloride, 0.5% of bovine fetal albumin, 0.5% of Ficoll, 0.5% of polyvinylpyrrolidone and 0.5% of sodium laurylsulfate) at 37°C for 2 hour, a probe labeled with ³²P was added thereto and hybridization was conducted at 42°C for 16 hours. After the filter was washed with washing solutions (with a 30 mM of sodium citrate (pH 7.0), 300 mM of sodium chloride and 0.1% of sodium laurylsulfate solution at room temperature for 5 minutes for two times and with a 15 mM of sodium citrate (pH 7.0), 150 mM of sodium chloride and 0.1% of sodium laurylsulfate solution at 60°C for 1 hour for two times), it was exposed to an X-ray film, the area which was sensitized with radioactive ray was compared with the plaque on the incubation dish and a positive clone was isolated from the plaque. The isolated positive λ phase clone was converted to a plasmid pBluescript which is a vector suitable for DNA sequence using a Helper Phage R408 of Stratagene and a nucleotide sequence was determined by a Sanger method. As a result thereof, 13 positive clones were able to be prepared. All of six among the above were clones containing almost the full length of the CRSP precursor cDNA while all of other six were clones containing almost the full length of the CRSP-2 precursor cDNA (Fig. 20). A remaining one was a short clone coding for a 3'-untranslated region of CRSP-3.

### Example 17 (Cloning of CRSP-3 and CT-2 cDNA)

A PCR where pig hypothalamus cDNA (corresponding to 20 ng of mRNA) was a template was carried out using primers (CRSP-3: GCCCAGCTTACGTCTCCTTT and TCAGGTAACTGCAATGATTT; CT-2: AGCAGCTTTGATTCTGCCAC and ACCTCCTCTCTGATATTCCA) and Pyrobest DNA polymerase of Takara Shuzo for 30 cycles each comprising at 94°C for 15 seconds, at 55°C for 15 seconds and at 72°C for 1 minute. The amplified DNA was subjected to an agarose gel electrophoresis, bands stained with ethidium bromide were recovered from agarose gel and subcloned into pBluescript II of Stratagene and a nucleotide sequence was determined by a Sanger method.

Analysis of a nucleotide sequence of the amplified DNA was conducted using a primer of CRSP-3 and, as a result, it was found to code for CRSP-3 (Fig. 5). On the other hand, in the PCR using a primer of CT-2, no amplification of DNA was observed.

### Example 18 (High-sensitivity quantification of expressed amount of gene of CRSP, CRSP-2, CRSP-3, CT-2, CT, CGRP and GAPDH by RT-PCR)

A template cDNA was prepared using total RNA (4 µg) of each tissue of pig, oligo dT primer and a Revertra Ace reverse transcriptase kit of Toyobo and 1/40 thereof was used for RT-PCR. The PCR for amplification of each genetic cDNA was conducted using rTaq polymerase of Toyobo in 30 cycles each comprising at 94°C for 15 seconds, at 60°C for 15 seconds and at 72°C for 1 minute and, with regard to the primer, the following sequences were used.

CRSP: CTCTCTGAGGAGGAATCACG and GAGTTCAGAGTCATAGTAACC

CRSP-2: CTCACAGAGGAGGAAGTCTC and TAGAGTTCAGTTCCTTGGTG

CRSP-3: AGCAGCTTTGATTCTGCCAC and TGCAGTGAAAGCAACTTGAG

CT-2: AGCAGCTTTGATTCTGCCAC and ACCTCCTCTCTGATATTCCA

CT: GCCACTCAGTGAGAAGGAAG and TGAGGCATGAGGGATGAAGC

CGRP: GCCACTCAGTGAGAAGGAAG and TCACCTTACATGTGTCCCCA

GAPDH: TCACTGCCACCCAGAAGACT and AGTGGTCGTTGAGGGCAATG

The amplified DNA was subjected to electrophoresis using 3% agarose gel and analyzed using Fuji Film FLA 2000. As a result, expression was observed in the central nervous system, thyroid gland and ovary for CRSP-2 and CRSP-3. On the other hand, no band was amplified in any tissue and no expression was observed for CT-2 (Fig. 24). It is believed that CT-2 was expressed limitedly in the areas other than the tissues shown in Fig. 24.

### Industrial Applicability

The present invention provides novel and useful peptides which are expressed in central nerves and strongly act on calcitonin receptor. The peptides of the present invention have a high homology to calcitonin gene-related peptides and have stronger action than calcitonin. They are also abundantly expressed in central nervous system and are very useful peptides as novel calcitonin-like peptides for osteoporosis and as analgesic agent, etc.

## Claims

1. A peptide which has at least the following amino acid sequence Ser-Cys-Asn-Thr-Ala-Thr-Cys-Met-Thr-His-          10 Arg-Leu-Val-Gly-Leu-Leu-Ser-Arg-Ser-Gly-        20 Ser-Met-Val-Arg-Ser-Asn-Leu-Leu-Pro-Thr-          30 Lys-Met-Gly-Phe-Lys-Val-Phe-Gly 38 or an amino acid sequence where a part of the amino acids are deleted, substituted or added and has properties of (1) being expressed in central nervous system, (2) strongly acting on calcitonin receptors and (3) promoting the cAMP productivity of cells.

2. The peptide according to claim 1, wherein the peptide further has (4) a property of incorporation of sodium ion concentration-dependently, (5) a property of suppressing the incorporation of calcium ion and (6) a property of suppressing the cell proliferation.

3. The peptide according to claim 1 or 2, wherein the peptide has at least an amino acid sequence shown in SEQ ID NO: 1, SEQ ID NO: 2, SEQ ID NO: 6, SEQ ID NO: 9, SEQ IS NO: 12, SEQ ID NO: 16 or SEQ ID NO: 19 of the Sequence Listing or an amino acid sequence where a part of amino acids are deleted, substituted or added.

4. The peptide according to any of claims 1 to 3, wherein the peptide is a peptide derived from mammals.

5. A gene which encodes the peptides according to any of claims 1 to 4.

6. The gene according to claim 5, wherein the gene has a nucleotide sequence shown in SEQ ID NO: 3, SEQ ID NO: 7, SEQ ID NO: 10, SEQ ID NO: 13, SEQ IS NO: 17 or SEQ ID NO: 20 of the Sequence Listing.

7. A pharmaceutical composition comprising the peptide according to any of claims 1 to 4 and a pharmaceutically acceptable carrier.

8. The pharmaceutical composition according to claim 7, wherein the pharmaceutical composition is a preventive/treating agent for osteoporosis, a preventive/treating agent for cancer, a diuretic agent, an appetite suppressing agent or an analgesic agent.

9. The pharmaceutical composition according to claim 7, wherein the pharmaceutical composition is a hypotensive agent or a drug which prevents restenosis after PTCA (percutaneous transluminal coronary angioplasty).


SEQUENCE LISTING

<110> Japan Science And Technology Agency
<110> Japan as represented by President of the National Cardiovascular

Center

<120> A New Peptide Having Production Activity of cAMP

<130> JA905066

<150> JP2002-162797
<151> 2002-06-04

<160> 22

<170> PatentIn Ver. 2.1

<210> 1
<211> 38
<212> PRT
<213> Swine

<220>
<221> modified amino acid
<222> (38)
<223> glycine amide

<220>
<223> CRSP

<400> 1
Ser Cys Asn Thr Ala Thr Cys Met Thr His Arg Leu Val Gly Leu Leu
  1               5                  10                  15

Ser Arg Ser Gly Ser Met Val Arg Ser Asn Leu Leu Pro Thr Lys Met
             20                  25                  30

Gly Phe Lys Val Phe Gly
         35


<210> 2
<211> 39
<212> PRT
<213> Swine

<220>
<223> CRSP-Gly

<400> 2
Ser Cys Asn Thr Ala Thr Cys Met Thr His Arg Leu Val Gly Leu Leu
  1               5                  10                  15

Ser Arg Ser Gly Ser Met Val Arg Ser Asn Leu Leu Pro Thr Lys Met
             20                  25                  30

Gly Phe Lys Val Phe Gly Gly
         35


<210> 3
<211> 679
<212> DNA
<213> Swine

<220>
<223> CRSP cDNA

<400> 3
tcaagtgtct ctgccgcttc ttccacagtg ccatcgcctg acgccaacgc tgctgcctct 60
gctccctcct ctgctccagt ccacctggtt cctgctgccc gaggggcacc atgggcttct 120
ggaaatttcc gcccttcctg gttctcagca tcctggtcct gtaccaggca ggcatgttcc 180
acacagcacc aatgaggtct gcctttggga gcccttttga tcctgctacc ctctctgagg 240
aggaatcacg cctccttttg gctgcaatgg tgaatgacta tgagcagatg aaggcccgtg 300
agatgcagaa gcagagggca cagggctccg gcatcagtgt ccagaagaga tcctgcaaca 360
ctgccacctg catgacccat cggctggtgg gcttgctcag cagatctggg agcatggtga 420
ggagcaacct gttgcccacc aagatgggct tcaaagtctt tggtgggcgc cgcaggaact 480
tttggatctg agcagtggga tgattccagg aggaaggtta ctatgactct gaactctatt 540
cgtttaattt acaatgaaag caacctacta aaaaatagca tggaagacat ccatgtatgc 600
atgcttctgg aaactgaaaa cactcttttc cttgaaataa actaaaacta aatgcaaaat 660
aaaatcaatg catcaatgc 679

<210> 4
<211> 126
<212> PRT
<213> Swine

<220>
<223> precursor peptide of CRSP

<400> 4
Met Gly Phe Trp Lys Phe Pro Pro Phe Leu Val Leu Ser Ile Leu Val
1               5                   10                  15

Leu Tyr Gln Ala Gly Met Phe His Thr Ala Pro Met Arg Ser Ala Phe
            20                  25                  30

Gly Ser Pro Phe Asp Pro Ala Thr Leu Ser Glu Glu Glu Ser Arg Leu
        35                  40                  45

Leu Leu Ala Ala Met Val Asn Asp Tyr Glu Gln Met Lys Ala Arg Glu
    50                  55                  60

Met Gln Lys Gln Arg Ala Gln Gly Ser Gly Ile Ser Val Gln Lys Arg
65                  70                  75                  80

Ser Cys Asn Thr Ala Thr Cys Met Thr His Arg Leu Val Gly Leu Leu
                85                  90                  95

Ser Arg Ser Gly Ser Met Val Arg Ser Asn Leu Leu Pro Thr Lys Met
            100                 105                 110

Gly Phe Lys Val Phe Gly Gly Arg Arg Arg Asn Phe Trp Ile
        115                 120                 125

<210> 5
<211> 3796
<212> DNA
<213> Swine

<220>
<223> gene CRSP

<400> 5
ctcgaggatc ctgcctcttg tttcccacaa atcctgcctt cctgtgcttg attccagctg 60
cctgaatcag accccctgct tgggcacaga atcatcaacc tgctgcgcat taacctccca 120
aaccgcactt ggacatggta gtcttagggg accggggatg ccttgtaatg ctggactctg 180
ctctacaaag atcacatagc tggggatgga gagggatgtg agcctgcgaa accgaacagg 240
taaagtttac catgacgtca aactgtcctt aaattcctgc tcactttgcg tgtgtttttc 300
gttggtgccc accaacctcc ccaccccctc ccacccccgc catcaatgac ctcaatgcaa 360
atacaagtgg ggtggtcctg ttggatgctc caggttctgg acgcaagtag tgacacaatc 420
ctggggctca ggatctttcc tctcattggt tgcctggagc tctgggacca ccccagattc 480
agagcggcgg gaataagagc agctgctggt gcggggaagg gttagaggca ctacccacct 540

caagtgtctc tgccgcttct tccacagtgc catcgcctga cgccaacgct gctgcctctg 600
ctccctcctc tgctccagtc cacctggttc ctgctgcccg gtaagcccgg agattcctgc 660
taagctgtgg ttctgtttct ctctccctct cctcccttcc ctctctctcc attggatttt 720
cttagctgat ctcttttccc gtctcaaagt tcctgtccac ttctctctgg gtctcttcat 780
cctgtaatat gccttactgc gcaattcatt ctaggctcct ttcacaggta actctggatg 840
gtctcagttc ggggattccc tgctctactc ttcctgagct gagctgggct ccagtcttgt 900
ccccgcagca gacgtgctta ggtccgtgtt gggattttgg agctctccag gcacttcagg 960
gagaggagga tgcaggaata gctttgagca gaagaaactt tcatggatcc catctcctct 1020
tacctacaag gatcgctgga aatggggtcg ggacctggga cagtgcaaat gggtggcaaa 1080
taggtgcaat gactgagggg aaagtagcta ttaaacgcaa gccccagttg aaggttctgg 1140
gaactccccc tcccgcaccg ccaccccatt taatcttggg tcccaattta aggctgtacc 1200
agcttgtttc ttacagggtg ctctttgcca gagtatggag cagctggaca gtaaaatttg 1260
gttcttcagt ttctcaggga ttccaactgc agagatatgt cctcccaact ccccttcccc 1320
ccagccaggt ataagcaaaa atcaggcatc aggagagatg ctgatgggtt gcactatggg 1380
aaaagctgtg gtgacaggta ctgcgagtct gtcctccagg agtcccggcc aacaggttga 1440
aggtgagagt gtgggtgtgc tgggcagggg gctatggacg gagacctcct cacccagttg 1500
tcctgctagg cttctttgct aaaccaaaca tgttgcaggc tcactggatc ttccagcagt 1560
ccacttggct gaggaggaaa tgatggtgaa aggaaaggac acgagcagcc tgaagccagg 1620
aagccaggga gttggaggca gaggcaggag cagagcccag gtctgtgggc tcaatgaact 1680
tggaactgct acaggtggtg acattgttct tcccttgcag aggggcacca tgggcttctg 1740
gaaatttccg cccttcctgg ttctcagcat cctggtcctg taccaggcag gcatgttcca 1800
cacagcacca atgaggtaag acagccctgc caacaagcac actcacttga tgagaatgta 1860
atataaacgt gtatataaat ttattataag gtggctctgt agaacaatgg atagtgcctt 1920
gcgctcctat aagtttatca taagctttat gtgtacacaa agtttgtaaa tagacataag 1980
atatacagta ctcatgattg taaattttat ataacttatc aaacctcaca gcatgctttt 2040
ttgttttcat caaatatttg tacctttagc acacgtatat gctcatatta ccataattta 2100
agaaatggat tgtatccaat ttgccaaata ctttgctagt aaatttgtta ttaaatctga 2160
tatgggatct acacatctca tttttcacct tcattcaaac tgcattaagc taaaattatt 2220
ttcccattca aactatcaga aaccaggcaa cctggctgtt tatcctgggg aggggcaggc 2280
aggagatcag aacctgtttt taggcttgct tcccctcctt aggtctgcct ttgggagccc 2340
ttttgatcct gctaccctct ctgaggagga atcacgcctc cttttggctg caatggtgaa 2400
tgactatgag cagatgaagg cccgtgagat gcagaagcag agggcacagg gctccgggta 2460
aggttccctg cccaaggaca acagggcatc cctttcttcc tctggtcagg cccaggaagg 2520
catattttaa agtcactttt gagttttctg acccccctgg acatgtctgt gggatgatta 2580
tggcatttcc cctgacggcc taggattttc tgctgtgatg accttttcta gcagaaatac 2640
tcaaggttca ctggtcctct caaggcagta gtcttccatg acgattctgt cgtacagcac 2700
ctgcactcaa cctctcactg acgggccttt tctttcttta tcccacaaat cagcatcagt 2760
gtccagaaga gatcctgcaa cactgccacc tgcatgaccc atcggctggt gggcttgctc 2820
agcagatctg ggagcatggt gaggagcaac ctgttgccca ccaagatggg cttcaaagtc 2880
tttggtgggc gccgcaggaa cttttggatc tgagcagtgg gatgattcca ggaggaaggt 2940
gactgccctt tttgtacctt cgggtgggag gacagaggac tgggtattgc aggggtgcat 3000
tccacaccct aaccctctgt gagcgcatgg gggtaaaacc tccacatggc aaggtgccca 3060
caccagtgtc tggagaaagg actgataatc cctataactg aaacattggg ctctttctct 3120
ctgtttctcc agtctctccc tgtgacactg acatcatctg ccaggaaata tagaccctgt 3180
ttacttaaaa cactgttccc tgggtattaa ttggggtcca gctctagcat tagaatttga 3240
aaggtaatga ccctaccctt ttggagcata ccttacaatg ttatgaactt ggagcataga 3300
ctcggattca aatactgtgt ctgtcttcca ctaactgtga ccataggcaa gtatgcctct 3360
gagcctcagc ttctccttgt aacttgaagg caacaatagt atcctcaata taaaaattaa 3420
ttagtataac atatgacaag agcctgttaa ctaagaatta ataacattct gttacttttt 3480
tccctcctag gttactatga ctctgaactc tacttcgttt aatttacaat gaaagcaacc 3540
tactaaaaaa tagcatggaa gacatccatg tatgcatgct tctggaaact gaaaacactc 3600
ttttccttga aataaactaa aactaaatgc aaaataaaat caatgcatca atgcagttac 3660
cttgtgtgca tcttttgtgt atatgattct ataatatgat gcatgtctca ttaggtttaa 3720
tggtagcaaa tctggcccct gtcagccaac ctgttggtgg gggcagctct gctaaacctc 3780
agggtcacat gaattc 3796

<210> 6
<211> 40
<212> PRT
<213> Bos sp.

<220>
<223> BosCRSP

<400> 6
Ala Cys Asn Thr Ala Thr Cys Met Thr His Arg Leu Ala Gly Trp Leu
1               5                   10                  15

Ser Arg Ser Gly Ser Met Val Arg Ser Asn Leu Leu Pro Thr Lys Met
            20                  25                  30

Gly Phe Lys Ile Phe Asn Gly Pro
        35                  40


<210> 7
<211> 649
<212> DNA
<213> Bos sp.

<220>
<223> BosCRSP cDNA

<400> 7
tgtctctgcc acttctgcca gactgccact gcctgctgcc aaagctactg ctgctgctcc 60
ttcctctgct cccagccacc tggtgccggc tgtcagagag gtgtcatggg cttctggaag 120
ttccccccat tcttggtcct cagcatcctg gtcttgtacc aggcaggcat gtttcatgca 180
gcaccattca ggtctgtctt tgatgggcgt tttgatcctg ctaccctgga tgaggaggaa 240
tcgcgcctcc tactggctgc gatggtgaat gactacgagc agatgagggc ccgggagtcg 300
gagaaggctc agaagaccga gggctcccgc atccagaaga gagcctgcaa cactgccacc 360
tgcatgaccc atcgcctggc aggctggctg agcagatctg ggagtatggt gaggagcaac 420
ttgctgccga ccaagatggg tttcaagatc ttcaatgggc cccgcaggaa ctcctggttt 480
taaacagtga aatgacgctg ggaataaggt caccaggaag ctgaactcta cttttagttt 540
gcatgaaggc accttacaaa aaaagaaaat agcatggaag atacccatgt atgcatgctt 600
ctcgatattg aaaacattct tcttttccct gaaataaact aaatgcaga          649


<210> 8
<211> 125
<212> PRT
<213> Bos sp.

<220>
<223> precursor peptide of BosCRSP

<400> 8
Met Gly Phe Trp Lys Phe Pro Pro Phe Leu Val Leu Ser Ile Leu Val
1               5                   10                  15

Leu Tyr Gln Ala Gly Met Phe His Ala Ala Pro Phe Arg Ser Val Phe
            20                  25                  30

Asp Gly Arg Phe Asp Pro Ala Thr Leu Asp Glu Glu Glu Ser Arg Leu
        35                  40                  45

Leu Leu Ala Ala Met Val Asn Asp Tyr Glu Gln Met Arg Ala Arg Glu
    50                  55                  60

Ser Glu Lys Ala Gln Lys Thr Glu Gly Ser Arg Ile Gln Lys Arg Ala
65                  70                  75                  80

Cys Asn Thr Ala Thr Cys Met Thr His Arg Leu Ala Gly Trp Leu Ser
                85                  90                  95

Arg Ser Gly Ser Met Val Arg Ser Asn Leu Leu Pro Thr Lys Met Gly
            100                 105                 110

Phe Lys Ile Phe Asn Gly Pro Arg Arg Asn Ser Trp Phe
        115                 120                 125


<210> 9
<211> 38
<212> PRT
<213> Canis sp.

<220>
<223> CanisCRSP

<400> 9
Ser Cys Asn Ser Ala Thr Cys Val Ala His Trp Leu Gly Gly Leu Leu
1               5                   10                  15

Ser Arg Ala Gly Ser Val Ala Asn Thr Asn Leu Leu Pro Thr Ser Met
            20                  25                  30

Gly Phe Lys Val Tyr Asn
        35


<210> 10
<211> 686
<212> DNA
<213> Canis sp.

<220>
<223> CanisCRSP cDNA

<400> 10
tctgccacat ccacggtgcc atcgcctgac atcggacgcc aacactgcca cagctgccgc 60
cgcctgtgct ccgagccacc ggctgcctgc agacagagaa gcgtcatggg cttctggaag 120
ttctcccctt tcctggttct cggcatcctg gcgctgtacc aggtgggctt cctccaggca 180
gcaccattca ggtctgcttt ggaaaatcct ccagactctg gtgtgcgcaa tgaggaggaa 240
ttgcgcctcc tcctggctgc agtgatgaag gactatatgc agatgaagac tcatgagctg 300
gagcaggagc aggagactga gggctccagg gttgctgtcc agaagagatc ctgcaactct 360
gccacctgtg tggcccattg gctgggaggc ttgctgagca gagccggaag tgtggcaaac 420
accaacttgc tgcccaccag catgggcttc aaggtctaca atcgacgccg cagggaactt 480
aaggcttaag cagtgacatg accccaggaa gaaggtcacc atgaagtgaa ctctacttct 540
cttaacttct aatgaaaaca acttatagaa tgcagagcat ggaagacaca tacatatgca 600
tgcttactat taaaacattg tgtcttgttt gaaataaagt aaaactaaat aaagagaata 660
aaatcataaa aaaaaaaaaa aaaaaa                                      686


<210> 11
<211> 127
<212> PRT
<213> Canis sp.

<220>
<223> precursor peptide of CanisCRSP

<400> 11
Met Gly Phe Trp Lys Phe Ser Pro Phe Leu Val Leu Gly Ile Leu Ala
1               5                   10                  15

Leu Tyr Gln Val Gly Phe Leu Gln Ala Ala Pro Phe Arg Ser Ala Leu
            20                  25                  30

Glu Asn Pro Pro Asp Ser Gly Val Arg Asn Glu Glu Glu Leu Arg Leu
        35                  40                  45

Leu Leu Ala Ala Val Met Lys Asp Tyr Met Gln Met Lys Thr His Glu
    50                  55                  60

Leu Glu Gln Glu Gln Glu Thr Glu Gly Ser Arg Val Ala Val Gln Lys
65                  70                  75                  80

Arg Ser Cys Asn Ser Ala Thr Cys Val Ala His Trp Leu Gly Gly Leu
                85                  90                  95

Leu Ser Arg Ala Gly Ser Val Ala Asn Thr Asn Leu Leu Pro Thr Ser
            100                 105                 110

Met Gly Phe Lys Val Tyr Asn Arg Arg Arg Arg Glu Leu Lys Ala
        115                 120                 125

<210> 12
<211> 37
<212> PRT
<213> Swine

<220>
<221> modified amino acid
<222> (37)
<223> Leucine amide

<220>
<223> CRSP-2

<400> 12
Ser Cys Asn Thr Ala Ser Cys Val Thr His Lys Met Thr Gly Trp Leu
1               5                   10                  15

Ser Arg Ser Gly Ser Val Ala Lys Asn Asn Phe Met Pro Thr Asn Val
            20                  25                  30

Asp Ser Lys Ile Leu
        35

<210> 13
<211> 690
<212> DNA
<213> Swine

<220>
<223> CRSP-2 cDNA

<400> 13
ctcaagtgtc tctgccgctt cttccacagt gccatcgcct gacgccaacg ctgctgcctc 60
tgctccctcc tctgctccag tccacctggt tcctgctgcc cgaggggcac catgggcttc 120
tggaaatttc cgcccttcct ggttctcagc atcctggtcc tgtaccaggc aggcatgttc 180
cacacagcac ccgtgagatt gcctttggag agcagctttg attctgccac tctcacagag 240
gaggaagtgt cccttctact ggttgcaatg gtgaaggatt atgtgcagat gaaggccact 300
gtgctggagc aggagtcaga ggacttcagc atcactgccc aggagaaatc ctgcaacact 360
gctagctgtg tgacccacaa gatgacaggc tggctgagca gatctgggag cgtggctaag 420
aacaacttca tgcccaccaa tgtggactcc aaaatcttgg gctgacgccg cagagagcct 480
caggcctgag ctgtgaaatg actccacaaa gaaggtcacc aaggaactga actctatttc 540
ttttaatctg caatgaaagc aatttatttg aaaaatagca tggaaaacac acatatatgc 600
atgcttcttg cttgaaatac agcttttagc ttgaaataaa ctaaaactaa atgcagaata 660
aaatcattgc agctacctga aaaaaaaaaa                                  690

<210> 14
<211> 117
<212> PRT
<213> Swine

<220>
<223> precursor peptide of CRSP-2

<400> 14
Met Gly Phe Trp Lys Phe Pro Pro Phe Leu Val Leu Ser Ile Leu Val
1               5                   10                  15

Leu Tyr Gln Ala Gly Met Phe His Thr Ala Pro Val Arg Leu Pro Leu
            20                  25                  30

Glu Ser Ser Phe Asp Ser Ala Thr Leu Thr Glu Glu Glu Val Ser Leu
        35                  40                  45

Leu Leu Val Ala Met Val Lys Asp Tyr Val Gln Met Lys Ala Thr Val
50                  55                  60

Leu Glu Gln Glu Ser Glu Asp Phe Ser Ile Thr Ala Gln Glu Lys Ser
65                  70                  75                  80

Cys Asn Thr Ala Ser Cys Val Thr His Lys Met Thr Gly Trp Leu Ser
                85                  90                  95

Arg Ser Gly Ser Val Ala Lys Asn Asn Phe Met Pro Thr Asn Val Asp
            100                 105                 110

Ser Lys Ile Leu Gly
        115

<210> 15
<211> 7673
<212> DNA
<213> Swine

<220>
<223> gene CRSP-2

<400> 15
   ggatccacta gttctagata aaatggacaa atacctagaa acagaagacc taccaagatg 60
   gaaggatgaa gaaatagaaa attcaaatac acctatgact aggaaggaga atgaagcatt 120
   aatccaaaat cttccaacaa agaaaagccc tggatacgat ggcctcattg gtgaatagta 180
   ccagacattt aaagaaaacg aataccaatc cttgtcaaac ttttccaaaa acctgaagag 240
   aaaggacaca ccctaaccta ttctatgagg caggccaaca ttactctgat accaaagatg 300
   gagaaagatt ctgcaaggag aaaaccccta cagacaaaat cctttatgac atggatgtgg 360
   aaaccctcaa cagtatgcta gggaattgaa ttcagaagcg tattaaaggg atcctacaac 420
   atgaccaagt gggatgaatt tctggaatgc aaggatgatt caaaatatga aaattgatca 480
   aagtgttata tcacaataat ggaatgtagg gaaaaacaca cctgattatt tccactgata 540
   cagaaaatta tttagtaaaa ttcaatacct tttcaggatt aaaaacaaaa actaggtata 600
   gaaggagact gcctcagcac aatacaacta tatatgaaaa accaacacca acaccataat 660
   ccagggtgga aaactgaaag cttttcccct aagatctgga agaaaatgga aaaaaatttt 720
   taagaatttt cagacagatt tgggtctctg gtacactctg agaaatcatc ttttagaatt 780
   tttttttttt aaaaataagc acaagaattt catttaaaag aagggaaata acatagcctt 840
   cagagtttat caggaggtgt aatttttttt tccacactag attgtggcta cctgatgcta 900
   attttgaggt ttaaacataa tgaaataaga ttgtacagcc aagtgccagc tagtcatgga 960
   acttttacct cagtactgtt tagtgcttca gtcctaagaa gtttcaggga gggctgcgtg 1020
   caatacaagt aatcggtact tgctgaaggt ctaaaatttc gagtgcactt ggtaaatcag 1080
   ggatgggcgc agaggagact ggttctgtaa ctcagactag tgaaccctag aatttagaaa 1140
   gggtactttt gtgctccaag caaatcctgt tctacctaac taggtccaaa tgctctgcag 1200
   gctgtagtta gagccctctc atagcaggga gactgccttg gtgaatctgc cagaggaaat 1260
   gaatttccat tcacattcat tcaacaaaca ttgggcgagt gccacctcat gtgcaaaaca 1320
   tggtgctaag tgctaaagaa aagatgttgt tttgtaaact tacccgcagc tcagagccag 1380
   gacttcttgg aaagtcagag gacttgagga aggagttcat ctcagcccct ccctcactgg 1440
   agagactggc ttttctttcc aagtaaagct taaaactgct ggaggctaag ttagcaccct 1500
   ctgggggcag accctgattc ctgcctctca tccccagccc tttgtgtgtg ggcgccaaag 1560
   atttctgagt gaggaatgaa tgttggcttt gaacaggaaa ggcacaagtg gcagccaagg 1620
   gtagaatgct gagcctacaa attaacatag ttacaaattt gtcttctaaa ggagtcgttt 1680
   cttagccata gtgcagccac ctttgcattg atcaaaactg tggttcttcc aatgaaaaaa 1740
   gacatcccca gacacacata cttacaaatg atttcagaag attgataggt cggaaatctc 1800
   aggttttgga ttttatttgc aaaagcgttt tgcgcctgag ttttaaactt tttttttttt 1860
   tttttttttt tgtatttttt cacttctagg gcggcttcgg cggcatatgg aaattcccag 1920
   gctaggggtc taataggcgc catagccacc ggcctacgcc agagctactg caacgctgga 1980
   tccgagccgc atctgcaacc tacaccacaa ctcacggcaa tgccggatcg ttaacccact 2040
   gagcaaggcc agggatcgaa cccgcaacct catggttctt actcagattc gttaaccact 2100
   gcgccacgac ggccactcca acctaccaga ctcttaatta agtagcagag tccaatttac 2160
   atgccgcacc acatctgtta ccccgagtta gcgaacttgg tcttggaact aactcctcac 2220
   ggaaagccaa gccgagtact cataattata gtgctgaacc cccaaaccct ggtctggcct 2280
   gtgcacccaa tttttgcttg tagtagaaac caggatttac ggagcccgag cagtccgcca 2340
   tcctgaactc ttctctttct caccttgcct tcatcctgga gtgcacctgc cctctatgaa 2400
   ccagttttcc cgttcccttg gtctcccgat ccgttgtcta tcctgaggag agcgagatgc 2460
   aagcacccga ttccctagcc ccaatatttt attctcttgc gaaggagaaa agttgaataa 2520
   gggtatcttg taaatgagat gttccgagtc cagagagcac aaaccggcaa ggggaacaga 2580
   tgtgccgcga ggcaggtgtg cggaaagata tagagaaggc tcaggttcgg acctgtggct 2640
   caggtcacac tcatggcaga gttcggttta atttcggctc tgcctggggg aaccacttaa 2700
   ctggggtcct tgctgccctc caccggcccc cgatgctgtt gcagcgtttg ccgcgctgga 2760
   gggtctgtac aggctgctgc ggtttatcgc tgtgtgctca gacacggtga tcctgagcag 2820
   catccgaact ggattggggt agatgtgggc acagggctgg aatcacaggt cactggaaca 2880
   tcttggcaaa cagcagccgg aagcaagggg cagctgggca aatggttctg ggacattgat 2940
   gggcttagat gatgaatggt ggggctggag gtcggcttgg cggcttggga agcatctatg 3000
   ccgtgcacgt ccctgcccaa gcccagtagg gcaccatctt tccccatatg gtggaccgac 3060
   cacccagcgc gactccagac atccgcacag aggtggggat tgggcaaatg gatcgcgatc 3120
   gcacagaatc ccctctgcac ttccctggta agctcttctc gatccctccc tgggtggaga 3180
   gcaggtacat ggctactaat gataccactc cttgaagacg ggaatatgat gccccgttcc 3240
   aaaaattaat atattgaggt gctagaagac actagcccga tgatcttact acctagaaaa 3300
   ggcacagctg gaacaaagtt tccgtgtgac aaagactgtg atcctgcctc ttgtttccca 3360
   caaatcctgc cttcctgtgc ttgattccag ctgcctgaat cagaccccct gcttgggcac 3420
   agaatcatca acctgctgcg cattaacctc ccaaaccgca cttggacatg gtagtcttag 3480
   gggaccgggg atgccttgta acgctggact ctgctctaca aagatcacat agctggggat 3540
   ggagagggat gtgagcctgc gaaaccgaac aggtaaagtt taccatgacg tcaaactgtc 3600
   cttaaattcc tgctcacttt gcgtgtgttt ttcgttggtg cccaccaacc tccccacccc 3660
   ctcccacccc cgccatcaat gacctcaatg caaatacaag tggggtggtc ctgttggatg 3720
   ctccaggttc tggacgcaag tagtgacaca atcctggggc tcaggatctt tcctctcatt 3780
   ggttgcctgg agctctggga ccaccccaga ttcagagcgg cgggaataag agcagctgct 3840
   ggtgcgggga agggttagag gcactaccca cctcaagtgt ctctgccgct tcttccacag 3900
   tgccatcgcc tgacgccaac gctgctgcct ctgctccctc ctctgctcca gtccacctgg 3960
   ttcctgctgc ccggtaagcc cggagattcc tgctaagctg tggttctgtt tctctctccc 4020
   tctcctccct tccctctctc tccattggat tttcttagct gatctctttt cccgtctcaa 4080
   agttcctgtc cacttctctc tgggtctctt catcctgtaa tatgccttac tgcgcaattc 4140
   attctaggct cctttcacag gtaactctgg atggtctcag ttcggggatt ccctgctcta 4200
   ctcttcctga gctgagctgg gctccagtct tgtccccgca gcagacgtgc ttaggtccgt 4260
   gttgggattt tggagctctc caggcacttc agggagagga ggatgcagga atagctttga 4320
   gcagaagaaa ctttcatgga tcccatctcc tcttacctac aaggatcgct ggaaatgggg 4380
   tcgggacctg ggacagtgca aatgggtggc aaataggtgc aatgactgag gggaaagtag 4440
   ctattaaacg caagccccag ttgaaggttc tgggaactcc ccctcccgca ccgccacccc 4500
   atttaatctt gggtcccaat ttaaggctgt accggcttgt ttcttacagg gtgctctttg 4560
   ccagagtatg gagcagctgg acagtaaaat ttggttcttc agtttctcag ggattccaac 4620
   tgcagagata tgtcctccca actccccttc cccccagcca ggtataagca aaaatcaggc 4680
   atcaggagag atgctgatgg gttgcactat gggaaaagct gtggtgacag gtactgtgag 4740
   tctgtcctcc aggagtcccg gccaacaggt tgaaggtgag agtgtgggtg tgctgggcag 4800
   ggggctatgg acggagacct tctcacccag ttgtcctgct aggcttcttt gctaaaccaa 4860
   gcatgttgca ggctcactgg atcttccagc agtccacttg gctgaggagg aaatgatggt 4920
   gaaaggaaag gacacgagca gcctgaagcc aggaagccag ggagttggag gcagaggcag 4980
   gagcagagcc caggtctgtg ggctcaatga acttggaact gctacaggtg gtgacattgt 5040
   tcttcccttg cagaggggca ccatgggctt ctggaaattt ccgcccttcc tggttctcag 5100
   catcctggtc ctgtaccagg caggcatgtt ccacacagca cccgtgaggt aagacagcac 5160
   tggtggcagt gctctcgctt cccacggccc ccggaatcat atagttctgt attgtgagtt 5220
   gtgctgtggt gagtctggct cttggtgggc ttctgtgtat agggggtgtg gggtcctaat 5280
   gtatgaatat agtcatgtat ataagtttat tataaatatt ttgtgatcca agataatatc 5340
   acaaagttta caaataaata gaagatatac agtattcact ataaatttct aaactcactg 5400
   aaccttacag catgtttttg ttgcttttta tgaaatgttt ataactttag caaacctata 5460
   tagtaattta gccataattt gagcaatgaa ttgcattcta attaagtaat ttgtcaataa 5520
   atttgttatt aaatctgaaa ggtaatctat acaatttctc accctctttc aaattatatt 5580
   aatatgaaac cattttcata ttcaaactat catttaattt ttaataatgg ctgtatttaa 5640
   cactaagctc atacaattcc tgaagatcta accatcagct ttcaaaagcc tacatgatgc 5700
   actttcagca gaactacttt gtggacaccc cagagcctaa ctcatggtga agcagcattt 5760
   ttggatgaac actagcctta tgtcctgacc gttgagaatt tcatcagcct tattctcaga 5820
   ggaagtggca gaaaccagga aatctggctg cttatcctag ggctgtggta ggctcagagc 5880
   gcatgttggg cttgctttcc cttcccagat tgcctttgga gagcagcttt gattctgcca 5940
   ctctcacaga ggaggaagtg tcccttctac tggttgcaat ggtgaaggat tatgtgcaga 6000
   tgaaggccac tgtgctggag caggagtcag aggacttcag gtcagtcttt gcacccctcc 6060
   cagaatatgg cttaccctct ccctagagta ccaggaaggc atatccttaa gaatgagatt 6120
   tgttatagtg ccataagcct tgatgtccag tctcataagc cttggtttat ttttagttta 6180
   ttacacagga gagattgtct attacagttc tgatttccag gtccagtaat gcagagccac 6240
   ctttgggttt tctgacaccc ctgaaaatgt ctatggggag tgatgatgca ttttcccaaa 6300
   agccctatgg ttttctgttg ggattttgtg tttagcagaa acatttcagg ttcactggtc 6360
   cctctcagag ctgtaatttt ccactgatgg tcagtcctgg ggggaatcac ttgccctcaa 6420
   gctgtcattg gcaggccttc tctttgtctc catcctgaaa atcagcatca ctgcccagga 6480
   gaaatcctgc aacactgcta gctgtgtgac ccacaagatg acaggctggc tgagcagatc 6540
   tgggagcgtg gctaagaaca acttcatgcc caccaatgtg gactccaaaa tcttgggctg 6600
   acgccgcaga gagcctcagg cctgagctgt gaaatgactc cacaaagaag gtgactgctc 6660
   tagaacatgg gatagcaggg caaatggctg ggtatttcag gggtgttggc tacactctaa 6720
   ccctccctga gcctgtactg taaaaaaaaa tccataatga agttgctgac cccattatcc 6780
   tcagaaagaa aagagaatcc taatagccaa aacccctata acttaggttc atttctattt 6840
   ttttccagtg tctcccagtg actctgaggt catctgtcag gaaacataga ttctattctt 6900
   ttttcttttc tttttggcta cacccaaggc atgtgaaagt ttttggccca gggattgaat 6960
   ctgaaccata gctgtgacct atgcagtacc tgtggcaaca ctggatcctt aacccaatgt 7020
   accacatcag gaactcctag gtcctattat ttaaaacact gttccctgca gttataattg 7080
   tgattattct agtttttgag tttgaaaggt aatgatctta tccagtgagt ttgaagtata 7140
   actacaatgt cacatatatc tgaattcaga gcattgactt ggtttcaaat gcgatgtctg 7200
   tcttccacta actatacaac catgggccag accctctctg aacctcagtt ctacatgaaa 7260
   ctttaaggca acaataatat ttacctgtta tcattaatat aaaaagtaac tgagataatt 7320
   catggtaaga gcctcactat taataagtaa taatattcta gctcttattt ttttttctcc 7380
   taggtcacca aggaactgaa ctctatttct tttaatctgc aatgaaagca atttatttga 7440
   aaaatagcat ggaaaacaca catatatgca tgcttcttgc ttgaaataca gcttttagct 7500
   tgaaataaac taaaactaaa tgcagaataa aatcattgca gctacctgat atgtatcatt 7560
   ttaatatttg attctgtatt ctataagtat gactcatgtc tcgctggctt atctggtagc 7620
   aaatctggac cctgtcagcc aacctgttgg tggtggcagc tctgctaaac ctc 7673

<210> 16
<211> 37
<212> PRT
<213> Swine

<220>
<221> modified amino acid
<222> (37)
<223> Leucine amide

<220>
<223> CRSP-3

<400> 16
Ser Cys Asn Thr Ala Ile Cys Val Thr His Lys Met Ala Gly Trp Leu
1               5                   10                  15

Ser Arg Ser Gly Ser Val Val Lys Asn Asn Phe Met Pro Ile Asn Met
            20                  25                  30

Gly Ser Lys Val Leu
        35

<210> 17
<211> 685
<212> DNA
<213> Swine

<220>
<223> CRSP-3 cDNA

<400> 17
gcccagctta cgtctccttt ctccgccagt gccatcacct gccaccagcg cggttgttgc 60
ttctcccact tgggctccaa gctacctggt tcctgcatcc agaggggcac catgggcttc 120
tggaagttcc cccccttcct gatcctcagc atcctggtcc tgtaccaagc aggaatgctc 180
catgccgcgc cattcaggat ggctttggga agcagctttg attctgccac actcacggaa 240
gaggaaatgt ccctcctact ggttgcaatg gtgaaggatt atgtgcagat gaaggccact 300
gtgctggagc aggagacaga ggacttcagc atcaccaccc aggagagatc ctgcaacact 360
gccatctgtg tgacccacaa gatggcaggc tggctgagca gatctgggag cgtggttaag 420
aacaacttca tgcccatcaa catgggctcc aaagtcttgg gccggcgccg cagacagcct 480
caggcctgag ctgtgaaatg actctaaaaa gaagttgaac tcaagttgct ttcactgcaa 540
agttgctttc cctgcaaatt aaaagaacca atttgaaaaa tagcatggaa gacacacata 600
tatgcatgct tcttgcttga aatacaactt tttgcttgaa acaaactaaa cctaaatgca 660
gaataaaatc attgcagtta cctga 685

<210> 18

<211> 125
<212> PRT
<213> Swine

<220>
<223> precursor peptide of CRSP-3

<400> 18
Met Gly Phe Trp Lys Phe Pro Pro Phe Leu Ile Leu Ser Ile Leu Val
1               5                   10                  15

Leu Tyr Gln Ala Gly Met Leu His Ala Ala Pro Phe Arg Met Ala Leu
            20                  25                  30

Gly Ser Ser Phe Asp Ser Ala Thr Leu Thr Glu Glu Glu Met Ser Leu
        35                  40                  45

Leu Leu Val Ala Met Val Lys Asp Tyr Val Gln Met Lys Ala Thr Val
    50                  55                  60

Leu Glu Gln Glu Thr Glu Asp Phe Ser Ile Thr Thr Gln Glu Arg Ser
65                  70                  75                  80

Cys Asn Thr Ala Ile Cys Val Thr His Lys Met Ala Gly Trp Leu Ser
                85                  90                  95

Arg Ser Gly Ser Val Val Lys Asn Asn Phe Met Pro Ile Asn Met Gly
            100                 105                 110

Ser Lys Val Leu Gly Arg Arg Arg Arg Gln Pro Gln Ala
        115                 120                 125


<210> 19
<211> 33
<212> PRT
<213> Swine

<220>
<221> modified amino acid
<222> (33)
<223> Serine amide

<220>
<221> modified amino acid
<222> (1)
<223> pyroglutamic acid

<220>
<223> CT-2

<400> 19
Glu Cys Asn Asn Leu Ser Thr Cys Val Leu Gly Thr Tyr Thr Trp Asp
1               5                   10                  15

Val Asn Lys Phe Tyr Ala Phe Pro Leu Thr Thr Thr Gly Ile Arg Val
            20                  25                  30

Ser



<210> 20
<211> 802
<212> DNA
<213> Swine

<220>

<223> CT-2 cDNA

<400> 20
gcccagctta cgtctccttt ctccgccagt gccatcacct gccaccagcg cggttgttgc 60
ttctcccact tgggctccaa gctacctggt tcctgcatcc agaggggcac catgggcttc 120
tggaagttcc cccccttcct gatcctcagc atcctggtcc tgtaccaagc aggaatgctc 180
catgccgcgc cattcaggat ggctttggga agcagctttg attctgccac actcacggaa 240
gaggaaatgt ccctcctact ggttgcaatg gtgaaggatt atgtgcagat gaaggccact 300
gtgctggagc aggagacaga ggacttcagc ctggacagct ccagagctaa gcagtgcaat 360
aatctgagta cctgtgtgct gggaacatat acatgggacg tcaacaagtt ttatgcattc 420
cccttaacta caactgggat tagagtatct ggcaagaaat gggtcagggc cagagtctca 480
gagaaagtcc attatccctc aaggcagcat accctaaggt gcttaagaag gcccccaccc 540
ctcctccttt ctagttcctc tcctagaatt tgcatgtgtt cttctctggt tgctctctga 600
gctgctatca gcagctttcc ttgtggccat ggatgtctgg aatatcagag aggaggtggg 660
gggtgggggc aggcaggcca gaagaaaatc actcaggaat agattaggag agaatgggca 720
gccctgtgag tgcctgtgga tttcacagca gagcttctca gtcctgcttc tgaacatgct 780
tttcactagg gaataaaagt at 802

<210> 21
<211> 162
<212> PRT
<213> Swine

<220>
<223> precursor peptide of CT-2

<400> 21
Met Gly Phe Trp Lys Phe Pro Pro Phe Leu Ile Leu Ser Ile Leu Val
1               5                   10                  15

Leu Tyr Gln Ala Gly Met Leu His Ala Ala Pro Phe Arg Met Ala Leu
            20                  25                  30

Gly Ser Ser Phe Asp Ser Ala Thr Leu Thr Glu Glu Glu Met Ser Leu
        35                  40                  45

Leu Leu Val Ala Met Val Lys Asp Tyr Val Gln Met Lys Ala Thr Val
    50                  55                  60

Leu Glu Gln Glu Thr Glu Asp Phe Ser Leu Asp Ser Ser Arg Ala Lys
65                  70                  75                  80

Gln Cys Asn Asn Leu Ser Thr Cys Val Leu Gly Thr Tyr Thr Trp Asp
                85                  90                  95

Val Asn Lys Phe Tyr Ala Phe Pro Leu Thr Thr Thr Gly Ile Arg Val
            100                 105                 110

Ser Gly Lys Lys Trp Val Arg Ala Arg Val Ser Glu Lys Val His Tyr
        115                 120                 125

Pro Ser Arg Gln His Thr Leu Arg Cys Leu Arg Arg Pro Pro Pro Leu
    130                 135                 140

Leu Leu Ser Ser Ser Ser Pro Arg Ile Cys Met Cys Ser Ser Leu Val
145                 150                 155                 160

Ala Leu


<210> 22
<211> 7142
<212> DNA
<213> Swine

<220>

<223> gene of CRSP-3 and CT-2

<400> 22
taccgggccc cccctcgagg tcgacggtat cgataagctt gatatcgaat tcctgcagcc 60
cgggggatcc ttaaccaact gagggaggcc agggattgaa ctgcgccctc atggatacta 120
gtccggtttg ttacacctca gcaacgggaa cccccctggt cactttgaag agggttttca 180
tacagatcct cccacctccg ctatcagtga cctcaatgcg aatacaagtg cggtggttct 240
gttaaattct ccaggttccg gaagcaagta ccgacataat cctcttgggg ctcaggattt 300
ttcccctcat tggttgcctg gagttccagg accaccctgg attcacagca gcgggaataa 360
gagcagctgc tggtgctgag aggcattaga agcactgccc agcttacgtc tcctttctcc 420
gccagtgcca tcacctgcca ccagcgcggt tgttgcttct cccacttggg ctccaagcta 480
cctggttcct gcatccaggt aagtccaaag attctgctta gctgtgattc tatttcttct 540
ctccctctcc tcccctccat ttctctccca ttacttttcc ttgccggtct cagaggctct 600
atccatttcc ctcggagttc cttcatcact caatctatta cgcgatttat ctcggggtcc 660
tttaacaggt aacactaaac ggtctcagtt ccagacactg agtctggctc cagttttgcg 720
gtctcctgcc gcatatgtgc ttaggttaat ctcaggaagc tggagcttcc tcaagcaatg 780
cgggctgagc agaacgcaga atatctttgt gtagaccgtg agctcaggga aaccctcttg 840
tgcctagctg tctgcgggaa atagactcat tttggaatgg attcggaact tagaccaggg 900
gaagcgggtg gcaaatagat gcaaagactg aggggcaggg agccattcaa ctgcaagtcc 960
tagctgccag tttggggagt tttttggttg tttttttctc cccagtttaa gtcctgtgtc 1020
ctaatttaag attccaccag cttgtttttt acagggtact ctttgccaaa gtttgaagca 1080
gctgagcagt agaatttggt tcttcagttt ctcagtgatt ctaaccacag agatatgtcc 1140
tcccaatccc cctcccctgc cagctatgag caaaagcctg gttcctggaa gacaactctg 1200
ggttgcatta tgtgaaaagc tgtggtggct tactccgagt ttgtcctcca ggagtcttgg 1260
ccaagaggtt gcaggtgaga gagtgtgtgg tactggggag ggggcaatgg actgagacca 1320
cctcacccag atgttctggt aggtttcttt gctaaaccaa gcatgctgca agcgcactgg 1380
atatttcagc agtgcacttg gctgaggaga aaatgatggt gaactgaaat gatatgacca 1440
gcctgaagcc aagaaaccag ggagttagag gcagaggcag gagcagaatc cagggtctgt 1500
aggctcatag aacttggaac tcctacaggt ggtgacatta ttcttccctt gcagaggggc 1560
accatgggct tctggaagtt cccccccttc ctgatcctca gcatcctggt cctgtaccaa 1620
gcaggaatgc tccatgccgc gccattcagg taagccagcc ctgccaggag ccctctcacc 1680
tccctcaacc cctgaaatct tagagttctg tgttgagtgg tactatgctg aatatggctc 1740
tctgtggggt tgtgggggtg tggggtcctg atgtacgaat gtaaacttgt atacaagtta 1800
atcagaaatg ttttaggagg ctatgtatca caaagtttac aaataaacaa aatatatagt 1860
acttgttatt tcatataacg cactgaacct cacagcatgc tgctattgct ttttctcaaa 1920
catttgtacc tttagcaaac ctatttacta atttcaccat actttgagca gtgggttaca 1980
tcctaatctg ctaattactt taccaataaa tttgttatta aatgtgatat gtggtccata 2040
catctaattt cttaccctca ttcaaattat attaagataa atattttcat attcaaacta 2100
tcattatcat ttattgttta ataatggctg tatttaacac taagctcata cagttcctga 2160
aaattaccat cagctttcaa aagcctatat gatccacctc cagcaaacct cttcttttag 2220
gtcaccctag agcctaactc gtggtgaagc agcatttttg catgaatact ggcctcatgt 2280
cctgtctgtt gagtttggtt ggccacatcc tcagtggaag tggcagaaat gaggagtagg 2340
ggttggggta ggctcagcac atgttgggtt tgcttccctt ccccaggatg gctttgggaa 2400
gcagctttga ttctgccaca ctcacggaag aggaaatgtc cctcctactg gttgcaatgg 2460
tgaaggatta tgtgcagatg aaggccactg tgctggagca ggagacagag gacttcaggt 2520
cagtctctgc acccctccca gaatatggct taccctctcc cctggagtac caggaaggca 2580
tgcgcgtgtg catgcacatg cacactcaca cacacaggta ggagagagca cagctagaca 2640
ggcagcctgg ggcacaactc ttctacaggc tccactaaaa tcataggtca tgtggaagaa 2700
ccacagataa aaacattcct ttctgaaagc agtaggggaa gccgtgagat cacctacagt 2760
ggaaattttg tagcagtact tgggagacct cccagcactg gagtttagcc ttgtaaggta 2820
aagccaggga atgaatctca tgtatctcag gagattttag aaatttggct ccttctcgtt 2880
aacctgccca tgatattctt tcacacctgc aaggcatctt cattgcacat ttgcaaggtg 2940
aagccagagc acttagaaaa gatgagtcag gtagagcagt ttctgaaata ggtctgaggc 3000
ctttagatgt gtagaatctg tggagatgtg catgttctca tggggccaga cacctttctc 3060
cagtcccaac tctctgtgca ctgagtttac tgttcatacc agctcctgac cgagctgtac 3120
ctgggcagag gcatgtgctg cacctttatc cgctctaaga acctctgtgc agagcataaa 3180
ggtctgagca gcatgtggaa tgccagaaaa ggtcgtccct ccccaccaca gcccttcccc 3240
acatcgccct ggctcagtga acctctgcat cctcctaatg gaggatgcat gagccgccct 3300
cctgcttgcc ccctcctgcc tctgttccag gtcccccttc cctggtctaa ccttctgcat 3360
gactgccctt gggggcagcc ctggtgcatg gtattgtctg gcatgtcttt tccctgcagc 3420
ctggacagct ccagagctaa gcagtgcaat aatctgagta cctgtgtgct gggaacatat 3480
acatgggacg tcaacaagtt ttatgcattc cccttaacta caactgggat tagagtatct 3540
ggcaagaaat gggtcagggc cagagtctca gagaaagtcc attatccctc aaggcagcat 3600
accctaaggt gcttaagaag gcccccaccc ctcctccttt ctagttcctc tcctagaatt 3660
tgcatgtgtt cttctctggt tgctctctga gctgctatca gcagctttcc ttgtggccat 3720
ggatgtctgg aatatcagag aggaggtggg gggtgggggc aggcaggcca gaagaaaatc 3780
actcaggaat agattaggag agaatgggca gccctgtgag tgcctgtgga tttcacagca 3840
gagcttctca gtcctgcttc tgaacatgct tttcactagg gaataaaagt atgtttctaa 3900

aaacacctga gctatagtgg ccatgtcaca tgcttcatgg atacagagac ttgtctgtca 3960
agtagcctta gtcctgggta gctggagtca gggcatggtg ggtggtccct ggagcaacct 4020
caagttgcaa aatcaggagc actaaggaac aaaacaagca cctctgggac ttgatgctac 4080
aaactcactt cctcttgcag gaagacaggg gaactttcct ttttctaagg agtactcagt 4140
acctctgaat gggaggcacc ttccagacaa gtccttaaga atggggttgg gtgtgccacc 4200
aaaatctctc tatgtcttgg ttaaatttta gtttacttgt acagaaaaga ttggctgtta 4260
aagttctgtg ttccttggct tgttgcccag agcacatttg ggttttctga cacctctgga 4320
aatgtctatg gagagtgatg atggcatttc cccaaaagcc ctatggtttt ctgttgggat 4380
tttgtgttta gcagaaacat ttcaggttca ctggtccctc tcagagctat aattttccac 4440
ggatggtcag tcctgggggg aagcacctgc cctcaggctc tcactgacag gccttctctt 4500
tgtctccatc ctgaaaatca gcatcaccac ccaggagaga tcctgcaaca ctgccatctg 4560
tgtgacccac aagatggcag gctggctgag cagatctggg agcgtggtta agaacaactt 4620
catgcccatc aacatgggct ccaaagtctt gggccggcgc cgcagacagc ctcaggcctg 4680
agctgtgaaa tgactctaaa aagaaggtga ctgctctaga acctggggta gcagggcaaa 4740
tggctgggta ttgcagaggt gctggccaca ctctaacctt ccgtgggcct gtattataaa 4800
aacatccaca gcaaaagggc caaccctagt gtccagagaa agaacagggt cccaagagct 4860
gaaatcccta gaatttggaa tcacttctat ttttttcagt ttctcccagt gattctgaga 4920
tcatctgcaa ggaaatatag atcctatgaa tttaaaacac tgttccctgc acttacaatt 4980
gccattgtag gttttgagtt ttaaaggtaa tgatcctatc caatgagttt gaagtatatc 5040
gtacaatgtc acgtacacct gaattcagag catagacttg gtttcaaatg tgatgtctgt 5100
cctctactaa ctatatgacc atgggccagg ccctctctgc gtctcagcct ctacatgtaa 5160
ttttaaggca aaaacagtat ctacctgtta ttgttaatat aaaaagtaat tgagataatt 5220
catggcaaga gcctcaacat taataaataa taatatccta gctcttgttt tttttttctc 5280
ctaggtcacc aagaagttga actcaagttg ctttcactgc aaagttgctt tccctgcaaa 5340
ttaaaagaac caatttgaaa aatagcatgg aagacacaca tatatgcatg cttcttgctt 5400
gaaatacaac tttttgcttg aaacaaacta aacctaaatg cagaataaaa tcattgcagt 5460
tacctgatgt gtatcttttt aatatttgat tctgtattct gtaagtatga ctcatgtctc 5520
actggcttat ctggtagcaa atctggaccc tgtcagccaa cctgttggtg gtggcagctc 5580
tgctaaacct caggagcaca tgaaattgct gccctatggg tgtctgggga tgcacagaaa 5640
tgttgagcct cagtggaacc tttaaagaaa tggtcttgga attcccatca tagctcagtg 5700
gaagcaaatc tgaccagcat cgataaggat gccggtttga tccatggcct tgcccattgg 5760
gtcaaggata tgttgttgcc atgagctgtg gtataggtta caggcgcagc tcagatctgg 5820
cattgctgtg gctgtggtat aggccagcag ctgcagctcc gattcaaccg ctagcctggg 5880
aacctccatg tgctgcaggt gcggccctaa aaagacagaa aaaaagaaga gaaaaaaaaa 5940
tgttctcatt tgttcacttc atcaagccag aaaatgtatt ttcagtacac ttaaaaggag 6000
tccctgctgc tatttatgct gtttccccca taagaacctc agggacctgt gaacacttgg 6060
ttgaccaggt tgctcaaatg aggcaatatc gtgcttgggg tgggtcctca gtatcctgta 6120
ctctcagtgt ctagtgaaac atccttatgg gattagaatc ctctgcatct cagagagaca 6180
ttcacattct cagagggcac cctggttccc agccccagaa gttatctgtt ctctctctcc 6240
ttgactcagg tttgccctta tccatccctg cctttcctcc ccaacagctc ctctttacac 6300
atcctatagc ctgcaaaacc ctttagagca atggctcaca gcttgaaagg gtatcgcaga 6360
ctggcagacc tggaaaggtc ctcagatgcc atctaatcca actttttact cttgaagttg 6420
cagagaagga aaattatgtt gctcagggtc ctgcaataac tttatgagac atccctatat 6480
ttaagaaata ataattgagt gtctgctatc tctttgacac tatttaagct ccaggaatag 6540
agcaataaac agaacagaca aaaccccctg cattcatgga gcttatattc taactggaag 6600
agactgaggt gatacatgct ctggaattag aaacattcag cactggaagg aatctgggat 6660
gtcataaggg tataaaggct tatttgatat agctatttca tgggatcaac atccctggca 6720
tatgctggca atgctggtta ccatctgttg actatgactt ccaaggtgac tggacagcca 6780
gtttcattgg tggacagtca attaagtggg atattttcca ttagagaagt catcccctac 6840
tacctacaga ttatagttat ttcctaagaa gcacatagaa ttatgaccct tctcccattc 6900
attctgtagt catcacagta acccaacctt agccataaag gtagatcaga ccatgtctcc 6960
cttaggacag aatactctat tatagactcc ccttctcaca aagtaaacat tttaggcatt 7020
gcagtgtctt cttaatcaac tctacttggt cagagtatat gtattaaaat ttacttccaa 7080
atttggaatt ccctggtggt taaggatcca ctagttctag agcggccgcc accgcggtgg 7140
ag 7142